# EUROPEAN PATENT SPECIFICATION

(11) **EP 2 310 496 B1**
(45) Date of publication and mention of the grant of the patent: **02.11.2016**
(21) Application number: 09781881.9
(22) Date of filing: 14.08.2009
(51) Int. Cl.: C12N 9/02, C12N 9/12, C12N 15/82

(54) **TRANSGENIC PLANTS**
TRANSGENE PFLANZEN
PLANTES TRANSGÉNIQUES

(30) Priority: 15.08.2008 GB 0814927
(43) Date of publication of application: 20.04.2011
(73) Proprietor: British American Tobacco (Investments) Limited, London WCR 3LA (GB)
(72) Inventor: JONES, Louise, London WC2R 3LA (GB); LEACH, Gwendoline, London WC2R 3LA (GB); COATES, Steve, Southampton SO15 8TL (GB)
(74) Representative: D Young & Co LLP
(86) International application number: PCT/EP2009/060582
(87) International publication number: WO 2010/018234

(56) References cited:
- WO-A-96/29858
- SHAUL O ET AL: "THREONINE OVERPRODUCTION IN TRANSGENIC TOBACCO PLANTS EXPRESSING A MUTANT DESENSITIZED ASPARTATE KINASE OF ESCHERICHIA COLI" PLANT PHYSIOLOGY, AMERICAN SOCIETY OF PLANT PHYSIOLOGISTS, ROCKVILLE, MD, US, vol. 100, 1 January 1992 (1992-01-01), pages 1157-1163, XP002035527 ISSN: 0032-0889
- PARIS STEPHANE ET AL: "Mechanism of control of Arabidopsis thaliana aspartate kinase-homoserine dehydrogenase by threonine." JOURNAL OF BIOLOGICAL CHEMISTRY, vol. 278, no. 7, 14 February 2003 (2003-02-14), pages 5361-5366, XP002553433 ISSN: 0021-9258 cited in the application
- GEPSTEIN SHIMON ET AL: "Large-scale identification of leaf senescence-associated genes." PLANT JOURNAL, vol. 36, no. 5, December 2003 (2003-12), pages 629-642, XP002553434 ISSN: 0960-7412 cited in the application

## Description

The invention relates to genetic constructs used in the preparation of transgenic plants. The constructs can have the ability to cause plants to accumulate threonine in their leaves, particularly during leaf senescence. The invention extends to plant cells transformed with such constructs, and to the transgenic plants themselves. The invention also relates to methods of producing transgenic plants, and to methods of increasing the concentration of threonine in senescent plants. The invention also relates to harvested plant leaves, for example tobacco leaves, that have been transformed with the genetic constructs, and to smoking articles comprising such harvested plant leaves.

Shaul et al Plant Physiol (1992) 100, 1157-1163 teaches threonine overexpression in transgenic tobacco plants expressing a mutant desensitized aspartate kinase of *E*. *coli*.

A primary target in increasing the flavour of flue-cured tobacco is the production of the amino acid threonine. Accumulation of high levels of threonine in the leaves of mutant tobacco plants gives significant flavour and aroma benefits. Normally, however, threonine production is tightly regulated, in conjunction with the production of other amino acids of the aspartate family, namely methionine, lysine and isoleucine. Therefore, modification of the biosynthetic pathway which produces threonine is required to achieve the flavour and aroma benefits.

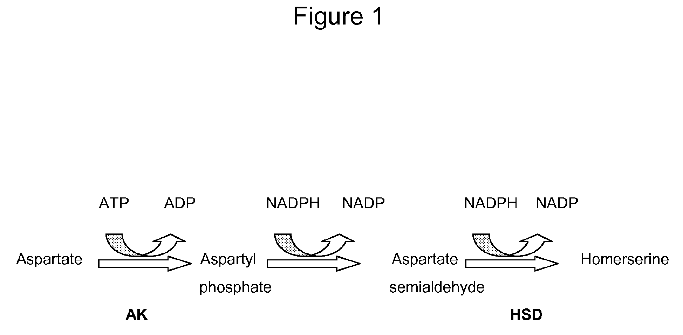

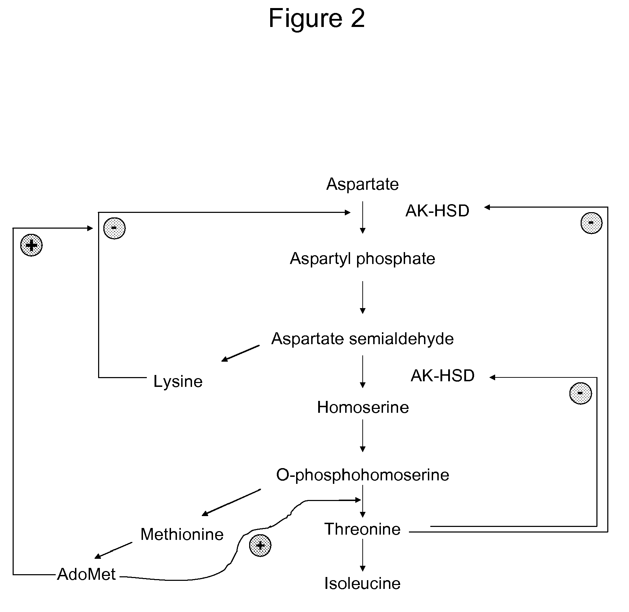

As shown in Figures 1 and 2, aspartate kinase (AK) is the first enzyme in the plant biosynthetic pathway which converts aspartate to amino acids including threonine. Endogenous aspartate kinase is regulated by feedback inhibition from both lysine and threonine. Therefore, there is a need for an aspartate kinase which is not subject to feedback inhibition. This needs to be achieved, however, without forcing the plant into methionine-starvation or depleting levels of aspartate to such an extent that the other pathways dependent on it are limited.

It has been previously shown that transgenic plants containing feedback-insensitive aspartate kinase, and which are able to overproduce threonine in comparison with wild-type plants, grew poorly and demonstrated a catastrophic fitness cost if such plants were homozygous for that mutation. Poor growth and a fitness cost is any non-beneficial change in the growth of the plants which the farmer notices. Clearly, any such change is not desirable.

The inventors of the present invention therefore set out to provide a transgenic plant which is able to accumulate threonine in leaves by overcoming the feedback inhibition loop discussed above, but ideally without any cost to its fitness. With this in mind, the inventors developed a number of genetic constructs, in which a gene encoding a threonine insensitive aspartate kinase (AK) enzyme was placed under the control of a senescence specific promoter, to determine what effect, if any, over-expression of this gene had on threonine levels in senescent leaves.

Leaf senescence is a phase of plant development during which the cells undergo distinct metabolic and structural changes prior to cell death. Physiological and genetic studies indicate that senescence is a highly regulated process. The progression of a leaf through senescence is visibly marked by the loss of chlorophyll and consequent yellowing, which results from the disassembly of the chloroplasts. The decreasing levels of leaf chlorophyll, characteristic of this developmental stage, can be measured, e.g. by solvent extraction and spectrophotometric measurement, or by a chlorophyll content meter. A decreased leaf chlorophyll level in comparison with an earlier leaf chlorophyll level recorded for the same plant, preferably grown under constant conditions, indicates senescence.

Molecular studies indicate that senescence is associated with changes in gene expression. The levels of mRNAs encoding proteins involved in photosynthesis decrease during senescence, whilst mRNA levels of genes encoding proteins thought to be involved in the senescence increase. Senescence is a highly organised process regulated by genes known as Senescence Associated Genes (SAGs). Leaf senescence involves the degradation of proteins, nucleic acids and membranes, and the subsequent transport of the nutrients resulting from this degradation to other regions of the plant, such as the developing seeds, leaves or storage organs. One problem of plant senescence is that many useful minerals and nutrients that are present in senescent leaves will remain in the leaves, and will therefore be effectively lost as the leaves die. For example, threonine, as well as many other amino acids, that are present in the senescent leaves, will go to waste, if they are not removed from the dying leaves.

As described in Example 2, the inventors carried out experiments which involved working with genetic constructs expressing threonine insensitive AK at specific locations within the plant, i.e. by use of the leaf-specific pea-plastocyanin promoter. However, they found that such transgenic plants had leaves which were pale, thickened, brittle and strap-like. The internodes were shortened and showed browning as maturity increased, and the buds either did not develop or were misshapen. Thus, such transgenic plants were unable to overcome the fitness cost.

Consequently, the inventors have developed a series of genetic constructs in which the feedback insensitive aspartate kinase (AK) activity is expressed only after the plant has entered senescence (under the control of the SAG12 promoter), and thus been allowed to develop normally. The inventors have observed that the constructs that they have developed, which allow transgenic plants transformed with these constructs to grow normally to maturity (i.e. to senescence) before the feedback insensitive aspartate kinase (AK) is switched on, are surprisingly able to overcome the fitness cost.

Therefore, according to a first aspect of the invention, there is provided a genetic construct comprising a senescence-specific promoter operably linked to a coding sequence encoding a polypeptide having threonine insensitive aspartate kinase activity.

The inventors have used a senescence-specific promoter linked to a coding sequence encoding a polypeptide having a threonine insensitive aspartate kinase activity to form the construct of the first aspect, which was then used to transform a plant. As a result of their studies, the inventors surprisingly found that the construct according to the invention, resulted in increased levels of threonine in senescent leaves. Furthermore, this temporal limitation on the expression of the transgene controlled by the senescence-specific promoter overcomes the negative effect of the fitness cost that was previously seen in earlier attempts to produce a threonine-accumulating plant. As shown in the Examples, the resulting transgenic plant produces threonine at a greater than wild-type level during leaf senescence. Threonine accumulation was demonstrated to occur in the leaves, and these increased levels are thought to positively contribute to the flavour of tobacco leaves containing the construct, and thus smoking articles made from such leaves.

The promoter in the genetic construct of the first aspect may be capable of inducing RNA polymerase to bind to, and start transcribing, the coding sequence encoding the polypeptide having threonine insensitive aspartate kinase activity.

A "senescence-specific promoter" (SAG) can be any promoter, which is associated with controlling the expression of a senescence-associated gene. Hence, the promoter can restrict expression of a coding sequence (i.e. a gene) to which it is operably linked substantially exclusively in senescing tissue. Therefore, a senescence-specific promoter can be a promoter capable of preferentially promoting gene expression in a plant tissue in a developmentally-regulated manner such that expression of a 3' protein-coding region occurs substantially only when the plant tissue is undergoing senescence. It will be appreciated that senescence tends to occur in the older parts of the plant, such as the older leaves, and not in the younger parts of the plants, such as the seeds.

One example of a plant which is known to express numerous senescence-associated genes is *Arabidopsis*. Hence, the promoter present in the construct according to the first aspect may be isolated from a senescence-associated gene in *Arabidopsis*. Gepstein et al. (The Plant Journal, 2003, 36, 629-642) conducted a detailed study of SAGs and their promoters using *Arabidopsis* as a model. Thus, the genetic construct may comprise a promoter from any of the SAG disclosed in this paper. For example, a suitable promoter may be selected from a group consisting of SAG12, SAG13, SAG101, SAG21, and SAG18.

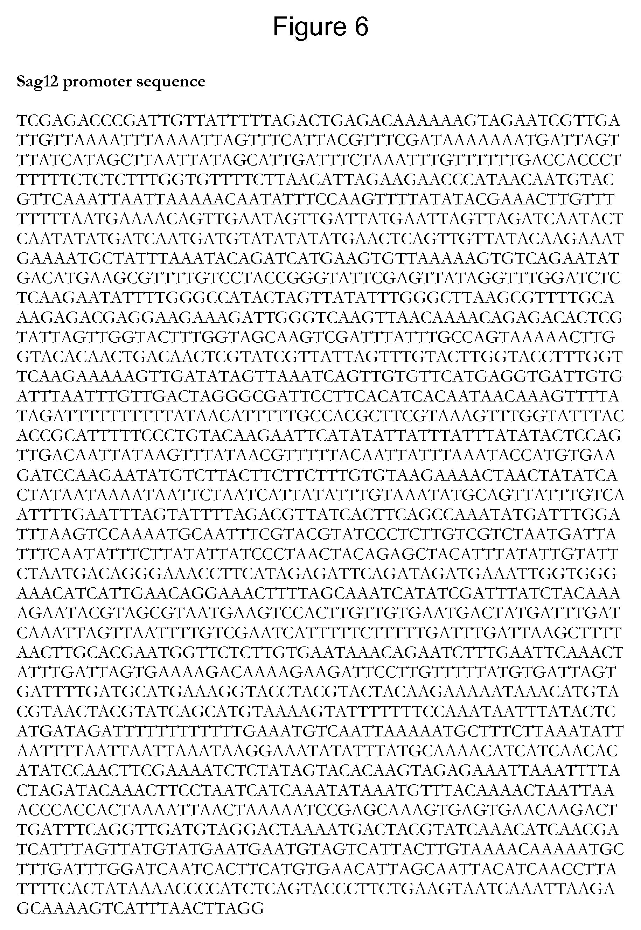

Preferred promoters are SAG12 and SAG13 promoters. In one embodiment, the promoter is a SAG12 promoter, which will be known to the skilled technician, (Gan & Amasino, 1997, Plant Physiology, 113: 313-319). The DNA sequence encoding the SAG12 promoter is shown in Figure 6, and is referred to herein as SEQ ID No.1, as follows: SEQ ID NO:1

Therefore, the promoter in the construct of the invention may comprise a nucleotide sequence substantially as set out in SEQ ID No.1, or a functional variant or functional fragment thereof wherein the variant or fragment thereof has at least 65% identity with SEQ ID No:1 and encodes a senescence specific promoter. The SAG12 promoter sequence may be obtained from *Arabidopsis thaliana*, as described in US 5,689,042. In embodiments where the promoter is SAG12, it will be appreciated that the promoter may comprise each of the bases 1-2093 of SEQ ID No:1. However, functional variants or functional fragments of the promoter may also be used in the genetic constructs of the invention.

A "functional variant or functional fragment of a promoter" can be a derivative or a portion of the promoter that is functionally sufficient to initiate expression of any coding region that is operably linked thereto. For example, in embodiments where the promoter is based on SAG12, the skilled technician will appreciate that SEQ ID No:1 may be modified, or that only portions of the SAG12 promoter may be required, such that it would still initiate gene expression, in the construct, of the polypeptide having threonine insensitive aspartate kinase activity. Similar modifications may be made to the nucleotide sequences of any of the other known SAG promoters, such as SAG 13, SAG101, SAG21 and SAG18.

Functional variants and functional fragments of the promoter may be readily identified by assessing whether or not transcriptase will bind to a putative promoter region, and then lead to the transcription of the coding sequence into the polypeptide having threonine insensitive aspartate kinase activity. Alternatively, such functional variants and fragments may be examined by conducting mutagenesis on the promoter, when associated with a coding region, and assessing whether or not gene expression may occur.

The genetic construct of the first aspect may be capable of causing, during senescence, expression of a polypeptide having threonine insensitive aspartate kinase activity. The promoter may induce expression of a coding sequence encoding a polypeptide exhibiting threonine insensitive aspartate kinase activity. Therefore, the genetic construct may comprise at least one coding sequence, which encodes threonine insensitive aspartate kinase (AK), or a functional variant or fragment thereof. Hence, in the first embodiment, the genetic construct may comprise the senescence-specific promoter and a coding sequence encoding threonine insensitive aspartate kinase (AK), or a functional variant or fragment thereof.

As described in Example 3-4, the inventors have found that expression of threonine insensitive aspartate kinase in a host cell, by transforming a plant with the construct of the invention, caused a significant increase in threonine levels. Furthermore, advantageously they found that the construct did not have any detrimental effect the fitness of the transformed plant.

As illustrated in Figures 1 and 2, in plants, the amino acids lysine, threonine, methionine and isoleucine are synthesised from aspartate. Several feedback inhibition loops have been found in this pathway. The first enzyme in the pathway, aspartate kinase (AK) (EC 2.7.2.4), catalyses the phosphorylation of aspartate to form 3-aspartyl phosphate with the accompanying hydrolysis of ATP. It is believed that higher plants generally possess at least two or three AK isozymes. AK activity undergoes negative feedback from the end product amino acids, lysine and threonine. At least one AK isozyme is feedback-inhibited by threonine and another by lysine and S-adenosyl methionine. In barley, AK activity can be separated into three isozymal peaks, one of which is inhibited by threonine and the other two by lysine. Additional feedback loops operate on other enzymes in the biosynthetic pathway such as dihydropicolinate synthase (DHPS) and homoserine desaturase (HSD).

As shown in Figures 1 and 2, Homoserine Desaturase (HSD) (EC 1.1.1.3), also known as Homoserine Dehydrogenase (HSDH), catalyses the first reaction that is uniquely associated with threonine, methionine and isoleucine biosynthesis, namely the conversion of 3-aspartic semialdehyde into homoserine. Higher plants generally possess at least two forms of HSDH: a threonine sensitive and a threonine insensitive form. Characterisations of purified HSDH and cDNA clones have confirmed that the HSDH isozymes are linked to AK on single proteins, and therefore denoted "AK:HSDH".

Elevating leaf threonine (Thr) content depends on overcoming the negative feedback control on the synthetic pathway. For example, transgenic tobacco expressing a lysine-insensitive AK from *E.coli* has been produced, which exhibited a threonine accumulating phenotype. In these transformants, the bacterial AK was expressed under the control of 35S in tobacco, either targeted to the cytoplasm or the chloroplast. The endogenous activity of AK was still entirely susceptible to inhibition by both lysine and threonine. The chloroplast-directed transgene gave higher AK activity. Higher threonine levels were found in the plants expressing the chloroplastic form. However, poor plant growth was noted and the homozygous plants demonstrated a fitness loss including wrinkled upper leaves, delayed flowering and partial sterility.

Work carried out in *Arabiclopsis* (Paris et al, 2003, The Journal of Biological Chemistry, Vol 278, no. 7, pp5361-5366) has demonstrated that the regulatory domain of the AK:HSDH enzyme contains two homologous sub-domains defined by a common loop-α helix-loop-β strand-loop-β strand motif. Site-directed mutagenesis was used to elucidate the threonine binding sites. It was found that each regulatory domain of the monomers of aspartate kinase-homoserine dehydrogenase possesses two non-equivalent threonine binding sites constituted in part by Gln⁴⁴³ and Gln⁵²⁴. The binding of threonine to Gln⁴⁴³ inhibits AK activity and also facilitates the binding of a second threonine to Gln⁵²⁴ which leads to inhibition of HSDH.

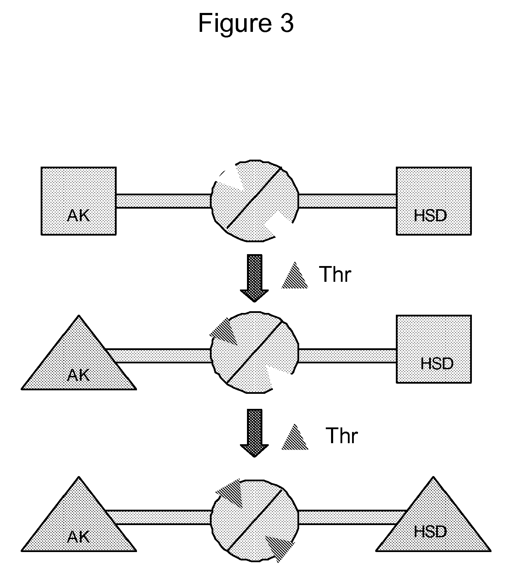

Figure 3 shows a proposed model for the inhibition of AK-HSDH by threonine in which the active catalytic domains of AK and HSDH are represented with squares and the inhibited catalytic domains are represented with triangles. Threonine (Thr) binding on the first sub-domain introduces both (i) conformational modifications of the other sub-domain, and (ii) conformational modifications of AK cathalytic domain leading to AK inhibition.

Conformational modification of the second sub-domain would induce the binding of a second threonine leading to conformational modifications of HSDH catalytic domain and HSDH inhibition.

The mutation of these glutamine residues to alanine rendered the threonine inhibition of the enzyme ineffective. The mutations do not affect the kinetics of the HSDH activity, only its sensitivity to threonine; the AK kinetics are only slightly modified. However, unfortunately, transgenic plants in which feedback insensitive AK:HSDH from *Arabidopsis* has been introduced and expressed also leads to a fitness cost.

In contrast, the genetic construct of the present invention causes expression of a polypeptide having threonine insensitive aspartate activity during senescence, and does not show any detrimental effect on the fitness of the transgenic plant. Thus, the genetic construct of the first aspect may encode a threonine insensitive aspartate kinase (AK), or a threonine insensitive bifunctional aspartate kinase-homoserine dehydrogenase enzyme (AK-HSDH), or functional variants or functional fragments thereof wherein the variant or fragment thereofhas at least 65% identity with SEQ ID NO:5, SEQ ID NO:6 or SEQ ID NO:7.

The threonine insensitive AK or bifunctional AK-HSDH, or functional variant or fragment thereof, may be derived from any suitable source, such as a plant. The coding sequence, which encodes the polypeptide having threonine insensitive aspartate kinase activity may be derived from *Arabidopsis spp., Zea spp., Flaveria spp.,* or *Cleome spp.*. The coding sequence, which encodes the polypeptide having threonine insensitive aspartate kinase activity may be derived from *Arabidopsis thaliana, Zea mays, Flaveria trinervia*, *Flaveria bidentis*, *Flaveria brownie* or *Cleome gynandra*. Preferably, the coding sequence of the enzyme may be derived from *Arabidopsis spp.,* such as *Arabidopsis thaliana.*

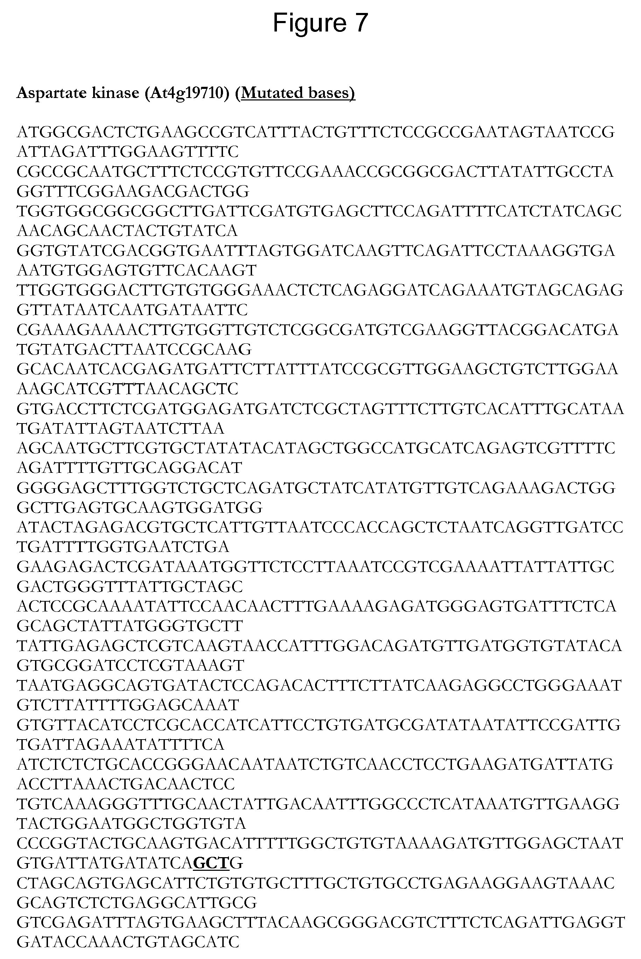

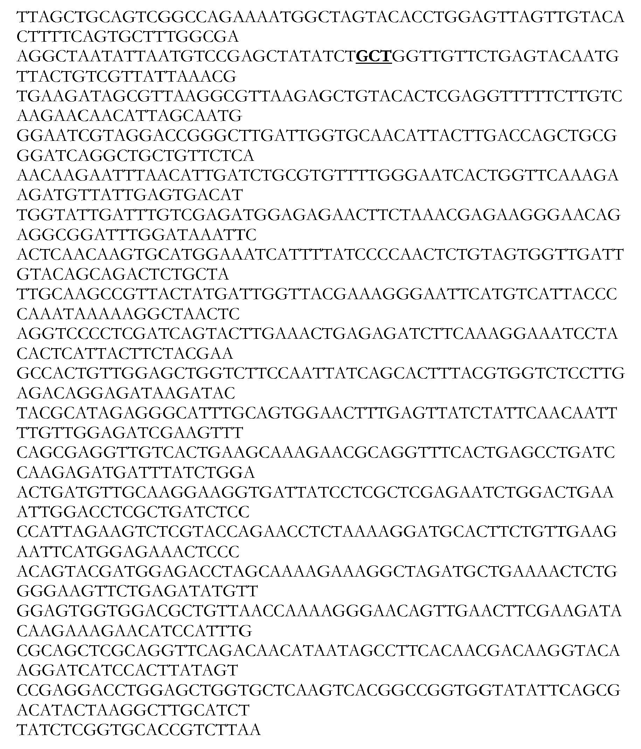

A particularly preferred threonine insensitive enzyme is a mutated AK-HSDH in which at least one of the threonine binding sites has been altered. Preferably, one or both of the threonine binding sites constituted in part by Gln⁴⁴³ and Gln⁵²⁴ have been mutated. For example, the *Arabidopsis* AK-HSDH may be mutated at Gln⁴⁴³ and/or Gln⁵²⁴. Preferably, the *Arabidopsis* AK-HSDH is mutated at Gln⁴⁴³ and Gln⁵²⁴. The mutated AK:HSDH gene used in the present invention is shown in Figure 7 in which the mutated bases are underlined.

Accordingly, the DNA sequence encoding one embodiment (i.e. Gln443Ala single mutant) of *Arabidopsis* threonine insensitive aspartate kinase is provided herein as SEQ ID No:2, as follows: SEQ ID No:2

In SEQ ID No:2 (i.e. Q443A), GCT, as highlighted, corresponds to mutated Gln⁴⁴³ encoding alanine, and CAR, as highlighted, corresponds to wild-type Gln⁵²⁴, where R may be either G or A.

The DNA sequence encoding another embodiment (i.e. Gln524Ala single mutant) of *Arabidopsis* threonine insensitive aspartate kinase is provided herein as SEQ ID No:3, as follows: SEQ ID No:3

In SEQ ID No:3 (i.e. Q524A), GCT, as highlighted, corresponds to mutated Gln⁵²⁴ encoding alanine, and CAR, as highlighted, corresponds to wild-type Gln⁴⁴³, where R may be either G or A.

The DNA sequence encoding another embodiment (i.e. Gln443Ala; Gln524Ala double mutant) of *Arabidopsis* threonine insensitive aspartate kinase is provided herein as SEQ ID No:4, as follows: SEQ ID No:4

In SEQ ID No:4 (i.e. Q443A; Q524A), GCT, as highlighted, correspond to mutated Gln⁴⁴³ and Gln⁵²⁴ both encoding alanine.

Accordingly, the coding sequence, which encodes the polypeptide having threonine insensitive aspartate kinase activity, may comprise a nucleic acid sequence substantially as set out in any one of SEQ ID No:2, 3 or 4, or a functional variant or a fragment thereof.

The polypeptide sequence of one embodiment (i.e. the Gln443Ala single mutant) of the threonine insensitive aspartate kinase is provided herein as SEQ ID No: 5, as follows: SEQ ID No:5

In SEQ ID No:5, the mutant Alanine (A) at position 443, and wild-type glutamine (Q) at position 524, are highlighted.

The polypeptide sequence of another embodiment (i.e. the Gln524Ala single mutant) of the threonine insensitive aspartate kinase is provided herein as SEQ ID No:6, as follows: SEQ ID No:6

In SEQ ID No:6, the mutant Alanine (A) at position 524, and wild-type glutamine (Q) at position 443, are highlighted.

The polypeptide sequence of another embodiment (i.e. Gln443Ala; Gln524Ala double mutant) of the threonine insensitive aspartate kinase is provided herein as SEQ ID No:7, as follows: SEQ ID No:7

In SEQ ID No:7, the mutant Alanine (A) at positions 443 and 524, are highlighted.

Accordingly, the polypeptide sequence having threonine insensitive aspartate kinase activity may comprise an amino acid sequence substantially as set out in any one of SEQ ID No:5, 6 or 7, or a functional variant or a fragment thereof wherein the variant or fragment has at least 65% identity with SEQ ID No:5, SEQ ID No:6 or SEQ ID No:7.

Genetic constructs of the invention may be in the form of an expression cassette, which may be suitable for expression of the coding sequence in a host cell. The genetic construct of the invention may be introduced in to a host cell without it being incorporated in a vector. For instance, the genetic construct, which may be a nucleic acid molecule, may be incorporated within a liposome or a virus particle. Alternatively, a purified nucleic acid molecule (e.g. histone-free DNA, or naked DNA) may be inserted directly into a host cell by suitable means, e.g. direct endocytotic uptake. The genetic construct may be introduced directly in to cells of a host subject (e.g. a plant) by transfection, infection, microinjection, cell fusion, protoplast fusion or ballistic bombardment. Alternatively, genetic constructs of the invention may be introduced directly into a host cell using a particle gun. Alternatively, the genetic construct may be harboured within a recombinant vector, for expression in a suitable host cell.

Hence, in a second aspect, there is provided a recombinant vector comprising the genetic construct according to the first aspect.

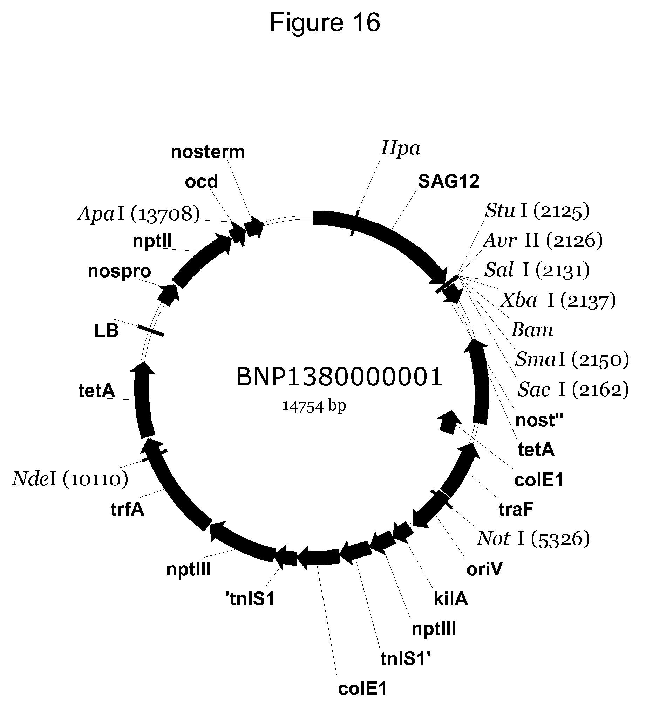

The recombinant vector may be a plasmid, cosmid or phage. Such recombinant vectors are highly useful for transforming host cells with the genetic construct of the first aspect, and for replicating the expression cassette therein. The skilled technician will appreciate that genetic constructs of the invention may be combined with many types of backbone vector for expression purposes. The backbone vector may be a binary vector, for example one which can replicate in both *E*. *coli* and *Agrobacterium tumefaciens.* For example, a suitable vector may be a pBIN plasmid, such as pBIN19. However, a preferred backbone vector is BAP1380000001, which is based on pBINPLUS (F. A. van Engelen et al. Transgenic Research (1995) 4, 288-290), and which harbours the SAG12 promoter. An embodiment of this vector is shown in Figure 16.

Recombinant vectors may include a variety of other functional elements in addition to the promoter (e.g. a senescence-associated promoter), and the at least one coding sequence (encoding mutated AK-HSDH). For instance, the recombinant vector may be designed such that it autonomously replicates in the cytosol of the host cell. In this case, elements which induce or regulate DNA replication may be required in the recombinant vector. Alternatively, the recombinant vector may be designed such that it integrates into the genome of a host cell. In this case, DNA sequences which favour targeted integration (e.g. by homologous recombination) are envisaged.

The recombinant vector may also comprise DNA coding for a gene that may be used as a selectable marker in the cloning process, i.e. to enable selection of cells that have been transfected or transformed, and to enable the selection of cells harbouring vectors incorporating heterologous DNA. Alternatively, the selectable marker gene may be in a different vector to be used simultaneously with vector containing the gene of interest. The vector may also comprise DNA involved with regulating expression of the coding sequence, or for targeting the expressed polypeptide to a certain part of the host cell, e.g. the chloroplast. Hence, the vector of the second aspect may comprise at least one additional element selected from a group consisting of: a selectable marker gene (e.g. an antibiotic resistance gene); a polypeptide termination signal; and a protein targeting sequence (e.g. a chloroplast transit peptide).

Examples of suitable marker genes include antibiotic resistance genes such as those conferring resistance to Kanamycin, Geneticin (G418) and Hygromycin (*npt-II, hyg*-B); herbicide resistance genes, such as those conferring resistance to phosphinothricin and sulphonamide based herbicides (*bat* and *suI* respectively; EP-A-242246, EP-A- 0249637); and screenable markers such as beta-glucuronidase (GB2197653), luciferase and green fluorescent protein (GFP). The marker gene may be controlled by a second promoter (which may or may not be a senescence-associated promoter), which allows expression in cells, which may or may not be in the seed, thereby allowing the selection of cells or tissue containing the marker at any stage of development of the plant. Suitable second promoters are the promoter of nopaline synthase gene of *Agrobacterium* and the promoter derived from the gene which encodes the 35S cauliflower mosaic virus (CaMV) transcript. However, any other suitable second promoter may be used.

Various embodiments of genetic constructs of the invention may be prepared using a suitable cloning procedure, which is described in Example 2, and which may be summarised as follows. The gene encoding wild-type AK-HSDH may be amplified from either the genomic or cDNA templates by PCR using suitable primers. Suitable primers for amplification of the wild-type AK-HSDH gene may be SEQ ID No:8 and/or SEQ ID No:9. PCR products may be examined using agarose gel electrophoresis. Site-directed mutagenesis using suitable pairs of primers may then be carried out in order to mutate the wild-type codons at position 443 and/or 524 to produce the Gln443Ala and Gln524Ala single mutants or the double mutant. For example, suitable primers for changing the codon for Gln⁴⁴³ may be SEQ ID No:10 and/or SEQ ID No:11. Suitable primers for changing the codon for Gln⁵²⁴ may be SEQ ID No:12 and/or SEQ ID No:3.

The PCR products encoding either of the two single mutants or the double mutant may then be ligated into a suitable vector for cloning purposes, for example that which is available under the trade name the pCR4 Blunt-TOPO vector, which may be obtained from Invitrogen. Vectors harbouring the PCR products may then be grown up in a suitable host, such as *E*. *coli. E. coli* colonies may then be screened by PCR using suitable primers, and inserts in plasmids showing the correct restriction enzyme digest pattern may be sequenced using suitable primers.

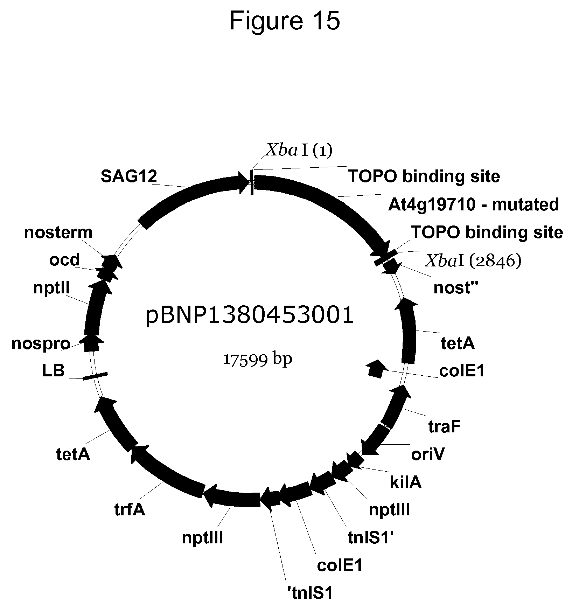

*E. coli* colonies carrying TOPO-cDNA (AK-HSDH) may be cultured to produce a suitable amount of plasmid, which may then be purified. The plasmid may then be digested to release a DNA fragment encoding mutant AK-HSDH, which may then be cloned into a vector harbouring a suitable promoter, for example a SAG promoter (preferably, SAG12), such as a pBNP plasmid. The resultant plasmid was named pBNP138-0453-001. An embodiment of the vector according to the second aspect may be substantially as set out in Figure 15.

In a third aspect, there is provided a method of producing a transgenic plant which accumulates a higher level of threonine in the leaves than a corresponding wild type plant cultured under the same conditions, the method comprising:
(i) transforming a plant cell with a genetic construct of the first aspect, or the vector of the second aspect; and
(ii) regenerating a plant from the transformed cell.

Methods for determining the level of threonine in plant leaves, and plant growth rates, are set out in Example 1. The method of the third aspect may comprise transforming a test plant cell with a genetic construct according to the first aspect, or a vector according to the second aspect. The genetic construct or the vector may be introduced into a host cell by any suitable means.

In a fourth aspect, there is provided a cell comprising the genetic construct according to first aspect, or the recombinant vector according to the second aspect.

The cell may be a plant cell. As the inventors have observed that expressing the threonine-insensitive aspartate kinase under the control of the senescence-specific promoter in a host cell is surprisingly effective at increasing threonine concentrations in senescent leaves without compromising fitness, the cell of the fourth aspect may comprise one or more constructs of the first aspect, or one or more vectors of the second aspect.

The cell may be transformed with the genetic construct or the vector according to the invention, using known techniques. Suitable means for introducing the genetic construct into the host cell may include use of a disarmed Ti-plasmid vector carried by *Agrobacterium* by procedures known in the art, for example as described in EP-A-0116718 and EP-A-0270822. A further method may be to transform a plant protoplast, which involves first removing the cell wall and introducing the nucleic acid, and then reforming the cell wall. The transformed cell may then be grown into a plant.

In a fifth aspect, there is provided a transgenic plant comprising the genetic construct according to first aspect, or the vector according to the second aspect.

The transgenic plant according to the fifth aspect may include the Brassicaceae family, such as *Brassica* spp.. The plant may be *Brassica napus* (oilseed rape).

Further examples of transgenic plants according to the fifth aspect include the family Poales, such as *Triticeae* spp. The plant may be *Triticum* spp. (wheat). Increasing the grain protein content in wheat may result in increased volume of food products comprising such wheat, such as bread.

Further examples of suitable transgenic plants according to the fifth aspect include the Solanaceae family of plants which include, for example jimson weed, eggplant, mandrake, deadly nightshade (belladonna), capsicum (paprika, chilli pepper), potato and tobacco. One example of a suitable genus of Solanaceae is *Nicotiana*. A suitable species of *Nicotiana* may be referred to as tobacco plant, or simply tobacco. Various methods for transforming plants with the genetic construct of the first aspect, or vector of the second aspect, are known and can be used in the present invention.

For example, tobacco may be transformed as follows. *Nicotiana tabacum* is transformed using the method of leaf disk co-cultivation essentially as described by Horsch et al. (Science 227: 1229-1231, 1985). The youngest two expanded leaves may be taken from 7 week old tobacco plants and may be surface sterilised in 8% Domestos^{™} for 10 minutes and washed 6 times with sterile distilled water. Leaf disks may be cut using a number 6 cork borer and placed in the *Agrobacterium* suspension, containing the appropriate binary vectors (according to the invention), for approximately two minutes. The discs may be gently blotted between two sheets of sterile filter paper. Ten disks may be placed on LS 3% sucrose + 2µM BAP + 0.2µM NAA plates, which may then be incubated for 2 days in the growth room.

Discs may be transferred to plates of LS + 3% sucrose + 2µM BAP + 0.2 µM NAA supplemented with 500 g/l claforan and 100 g/l kanamycin. The discs may be transferred onto fresh plates of above medium after 2 weeks. After a further two weeks, the leaf disks may be transferred onto plates containing LS + 3% sucrose + 0.5µM BAP supplemented with 500 mg/l claforan and 100 mg/l kanamycin. The leaf disks may be transferred onto fresh medium every two weeks. As shoots appear, they may be excised and transferred to jars of LS +3% sucrose supplemented with 500 mg/l claforan. The shoots in jars may be transferred to LS + 3% sucrose + 250 mg/l claforan after approximately 4 weeks. After a further 3-4 weeks the plants may be transferred to LS + 3% sucrose (no antibiotics) and rooted. Once the plants are rooted they may be transferred to soil in the greenhouse.

In a sixth aspect, there is provided a plant propagation product obtainable from the transgenic plant according to the fifth aspect.

A "plant propagation product" can be any plant matter taken from a plant from which further plants may be produced. Suitably, the plant propagation product may be a seed.

The present invention also embraces harvested leaves from a transgenic plant of the present invention in which the harvested leaves contain a higher level of threonine than harvested leaves from a corresponding wild type plant cultured under the same conditions.

Therefore, in a seventh aspect, there is provided a harvested leaf containing a higher level of threonine than harvested leaves than the corresponding level of threonine in a harvested leaf taken from a wild-type plant cultured under the same conditions, wherein the leaf is harvested from the transgenic plant according to the fifth aspect, or produced by the method according to the third aspect.

An eighth aspect of the invention provides a smoking article comprising threonine-enriched tobacco obtained from a mutant tobacco plant, which mutant is capable of over-producing threonine in senescent leaves.

Advantageously, and preferably, the mutant tobacco plant may have been transformed with a genetic construct or vector of the invention. The smoking article may be a cigarette, cigar, cigarillo, or rolling tobacco, or the like.

Threonine-reduced tobacco can include tobacco in which the threonine concentration is more than the corresponding concentration in a wild-type plant cultured under the same conditions. Such a smoking article may comprise tobacco obtained from a mutant tobacco plant, which may have been transformed with a genetic construct according to the first aspect of the invention, or a vector according to the second aspect. The flavour and aroma of the threonine-enriched tobacco is improved.

It will be appreciated that the present invention provides a method of increasing the level of threonine in plant leaves above the corresponding wild type level without compromising plant fitness, this comprises altering plant metabolism to achieve increased production of threonine after initiation of leaf senescence.

Hence, in a ninth aspect of the invention, there is provided a method of increasing the level of threonine in plant leaves above the corresponding wild type level without compromising plant fitness, the method comprising altering plant metabolism to achieve increased production of threonine after the initiation of leaf senescence, wherein the method comprises transforming the plant with the construct or vector according to the present invention. Advantageously, the methods according to the invention do not compromise the health or fitness of the plant that is generated. The methods comprise transforming the test plant, and preferably its leaves, with the genetic construct of the first aspect, or the vector of the second aspect.

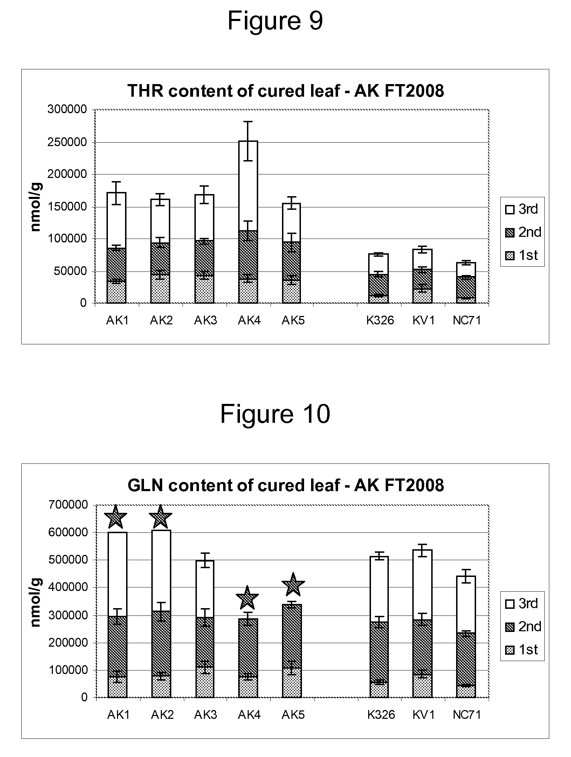

As described in Example 4, in addition to measuring threonine levels in the transformed plants of the invention, and showing that threonine concentration increase in senescent leaves, the inventors have also measured the concentrations of other amino acids in the transgenic plant, including glutamine, glutamic acid, aspartic acid and histidine. As shown in Figures 10-14, the concentrations of each of these amino acids were comparable or even higher than the controls, providing a strong indication that the fitness of the transgenic plant had not been compromised. In summary therefore, the construct of the invention may be suitably used to increase the level of threonine in senescent leaves without negatively affecting the fitness of the transgenic plant.

It will be appreciated that also disclosed is any nucleic acid or peptide or variant, derivative or analogue thereof, which comprises substantially the amino acid or nucleic acid sequences of any of the sequences referred to herein, including functional variants or functional fragments thereof. The terms "substantially the amino acid/polynucleotide/polypeptide sequence", "functional variant" and "functional fragment", can be a sequence that has at least 40% sequence identity with the amino acid/polynucleotide/polypeptide sequences of any one of the sequences referred to herein, for example 40% identity with the promoter identified as SEQ ID No:1 (i.e. SAG12 promoter) or the gene identified as SEQ ID No.2, 3 or 4 (which encode various embodiments of the AK-HSDH enzyme), or 40% identity with the polypeptide identified as SEQ ID No.5, 6 or 7 (i.e. various embodiments of the mutant AK-HSDH enzyme), and so on.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22, 4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula:-Sequence Identity = (N/T)*100.

Alternative methods for identifying similar sequences will be known to those skilled in the art. For example, a substantially similar nucleotide sequence will be encoded by a sequence which hybridizes to the sequences shown in SEQ ID No's: 1, 2, 3 or 4, or their complements under stringent conditions. By stringent conditions, we mean the nucleotide hybridises to filter-bound DNA or RNA in 3x sodium chloride/sodium citrate (SSC) at approximately 45°C followed by at least one wash in 0.2x SSC/0.1% SDS at approximately 20-65°C. Alternatively, a substantially similar polypeptide may differ by at least 1, but less than 5, 10, 20, 50 or 100 amino acids from the sequences shown in SEQ ID No: 5, 6 or 7.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will known the nucleotide sequences encoding these amino acids.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
Figure 1 shows part of the plant biosynthetic pathway in which aspartate is converted into other amino acids. The enzymes involved are aspartate kinase (AK) and homoserine desaturase (HSDH);
Figure 2 shows the plant biosynthetic pathway in which aspartate is converted into other amino acids including threonine. The first enzyme activity in the pathway is aspartate-kinase (AK). Plants can produce a bifuntional enzyme aspartate-kinase:homoserine dehydrogenase (AK-HSDH). The biosynthetic pathway is tightly regulated involving positive and negative feedback by the end products;
Figure 3 shows a model for the allosteric control of aspartate kinase-homoserine dehydrogenase by threonine in Arabidopsis (after Paris et al (2003));

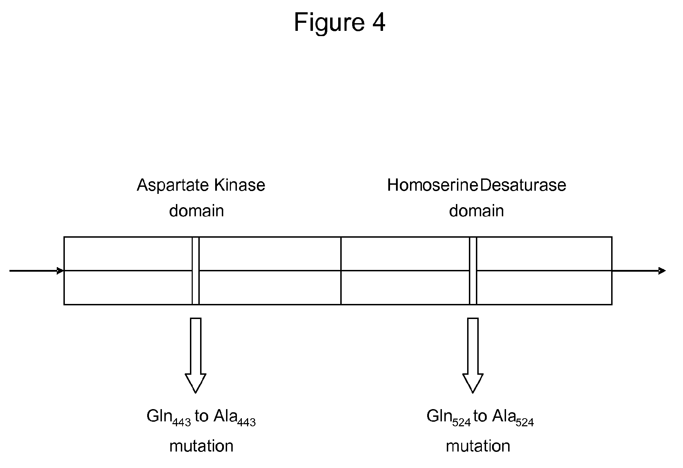

Figure 4 schematically shows a mutated bifunctional aspartate kinase homoserine desaturase enzyme;
Figure 5 shows the levels of threonine detected in leaf discs in Example 2;
Figure 6 shows the sequence of the SAG12 promoter;
Figure 7 shows the sequence of the mutated aspartate kinase homoserine desaturase enzyme. The mutated bases required to overcome feedback inhibition by threonine are shaded and underlined;
Figure 8 shows the amount of threonine in leaf disks taken from transgenic plants in which the threonine insensitive AK-HSDH is under the control of a senescence specific promoter as described in Example 3;
Figure 9 is a bar chart showing the content of threonine in cured leaf grown by field trial;
Figure 10 is a bar chart showing the content of glutamine in cured leaf grown by field trial;
Figure 11 is a bar chart showing the content of glutamic acid in cured leaf grown by field trial;
Figure 12 is a bar chart showing the content of asparagine in cured leaf grown by field trial;
Figure 13 is a bar chart showing the content of aspartic acid in cured leaf grown by field trial;
Figure 14 is a bar chart showing the content of histidine in cured leaf grown by field trial;
Figure 15 is a plasmid map of one embodiment of a vector according to the invention; and
Figure 16 is a plasmid map of a backbone vector used in accordance with the invention.

### Examples

As described in Example 2, the inventors have developed transgenic plants in which modified feedback insensitive aspartate kinase (AK:HSDH) was expressed under the control of a leaf-specific promoter. However, they found that localising expression of modified AK:HSDH to leaves resulted in a fitness cost to the transgenic plant. Therefore, as described in Examples 3-4, the inventors then used a senescence-specific promoter (SAG12) linked to a coding sequence encoding a polypeptide having a threonine insensitive aspartate kinase activity (AK:HSDH). The resulting transgenic plant produced threonine at a greater than wild type level during leaf senescence without compromising the plant's fitness.

### Example 1 - A test for threonine levels in leaves

The test for threonine levels was carried out on green or yellowing leaf. Leaf discs were taken and used for analysis so the measurements were based on the amounts of threonine per leaf disc (i.e. amount of thr/leaf area) or were related to the amount of protein in the supernatant (i.e. amount of thr/mg protein). The leaf disc was mashed with a set volume of water and centrifuged to sediment the insoluble leaf debris. Supernatant from this process was then processed using the Phenomenex EZfaast Kit. This is a proprietary kit which is used to derivatise the amino acids in the extract such that they can be quantified using a standard lc/ms set-up.

Calibration is by means of external standard for each amino acid to be quantified and the efficiency of the derivatisation steps is normalised between samples by the inclusion in the process of internal standards. The chromatograms are assessed by peak area and related to concentration using the integration algorithms in the lc/ms software. If no peak can be confidently identified by the software or operator, this is stated as "below limits of detection". This was found to be the case for some of the empty-vector controls and some of the segregating null plants in the next generations.

### Example 2 - Transgenic plants with feedback insensitive AK:HSDH operably linked to a leaf specific promoter

The inventors performed site-directed mutagenesis on the bifunctional AK:HSDH wild-type sequence from *Arabidopsis thaliana* (At4g19710). Hence, the wild-type sequence was first isolated from a leaf specific cDNA library from *Arabidopsis thaliana* by PCR using the following primers:
- At4g19710 For.: ATGGCGACTCTGAAGCCGTCATTTAC (SEQ ID No:8);
- At4g19710 Rev.: TTAAGACGGTGCACCGAGATAAGATGC (SEQ ID No:9).

The wild-type sequence was modified in one of three ways: (i) the AK domain only was mutated, (ii) the HSDH domain was mutated, or (iii) both domains were mutated to disallow regulation by Thr binding. The specific mutations were on Gln443 & Gln524, both in the enzyme regulatory domains, both of which were mutated by site-directed mutagenesis to Alanine (Paris et al (2003), "Mechanism of control of Arabidopsis thaliana aspartate kinase-homoserine dehydrogenase by threonine", J.Biol.Chem 278:5361-5366, and Frankard et al (1992), "Two feedback-insensitive enzymes of the aspartate pathway in Nicotiana sylvestris" Plant Physiol 99:1285-1293.

The Stratagene® QuikChange® Site-Directed Mutagenesis Kit (Catalog #200518) was used for this procedure. To change the codon coding for Glu443, the following primers were used in the site-directed mutagenesis reactions:
- Gln443 For.: GTGATTATGATATCAGCTGCTAGCAGTGAGCATTCTG (SEQ ID No:10); and
- Gln443 Rev.: CAGAATGCTCACTGCTAGCAGCTGATATCATAATTCAC (SEQ ID No:11).

For Gln524, the following primer pair was used:
- Gln524 For.: GTCCGAGCTATATCTGCTGGTTGTTCTGAGTACAATG; (SEQ ID No:12); and
- Gln524 Rev.: CATTGATCTCAGAACAACCAGCAGATATAGCTCGGAC (SEQ ID No:13).

These three mutant sequences were used individually to transform *Nicotiana tabacum* plants, as was the wild-type *Arabidopsis* AK:HSDH. In all cases, the gene of interest was expressed under the control of the leaf-specific pea-plastocyanin promoter. Plants of all populations were generated through *Agrobacterium-*mediated transformation and were grown under glasshouse conditions in Cambridge, UK. EZfaast amino acid kit (Phenomenex®) was used to extract and derivatise the free amino acids in each sample. Quantification was then carried out by LC/MS.

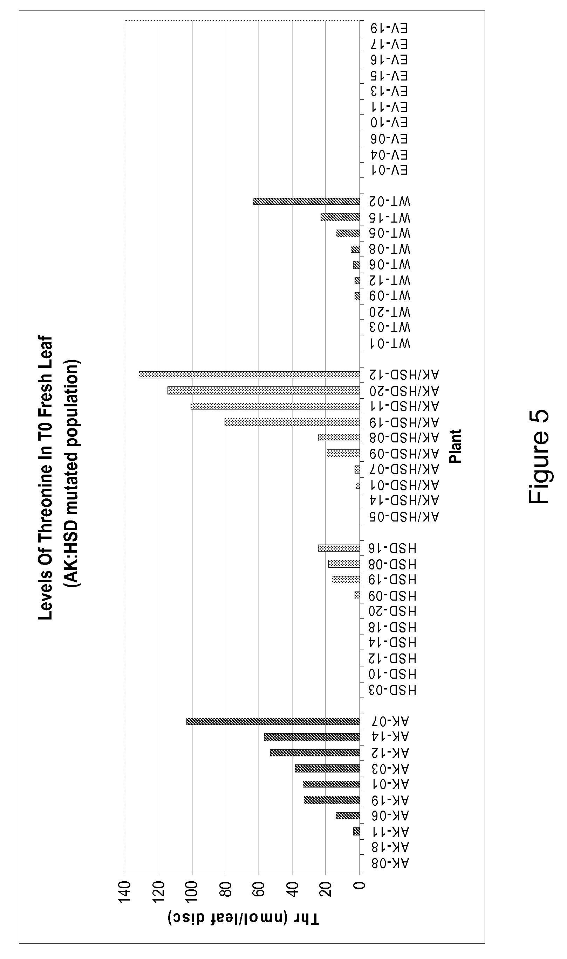

The results are shown in Figure 5 and in Table 1. In Figure 5, the series of plants in which the AK domain only was mutated have names starting AK, the series of plants in which with the HSDH domain only was mutated have names starting HSDH, the series of plants in which both AK and HSDH domains are mutated have names starting AK/HSDH, wild type plants have names starting WT and transgenic plants containing empty vector controls have names starting EV.

The inventors achieved elevated leaf threonine levels in all populations transformed with the *Arabidopsis* sequence - including that transformed with the non-mutated *Arabidopsis* sequence. The populations transformed with sequences mutated at the AK domain gave the highest leaf Thr levels. These correlated with the highest proportion of the populations showing severely compromised fitness. Although the inventors had used a leaf-specific promoter with the intention of reducing the impact of the modification on fertility, the effect that they had was sufficient that the entire plant was still affected by the metabolic consequences of de-regulating the feedback control on the enzyme.

However, in all plants showing elevated threonine, there was a correlation with altered growth habit. Leaves were pale, thickened, brittle and strap-like. Internodes were shortened and showed browning as maturity increased. Buds either did not develop or were misshapen. In conclusion, the site-directed mutagenesis was successful in providing elevated leaf threonine. However, the fitness costs of releasing the feedback control on the aspartate kinase still needed to be overcome if an agronomically viable plant with high leaf threonine was to result from this approach.

**Table 1**

| Individual (ranked on increasing Thr) | Threonine (nmol/leaf disc) | | | | |
|---|---|---|---|---|---|
| | **AK** | **HSD** | **AK/HSD** | **WT** | **Control** |
| 1 | bld | bld | bld | bld | bld |
| 2 | bld | bld | bld | bld | bld |
| 3 | 4.1a | bld | 2.5^{a} | bld | bld |
| 4 | 14.1^{b} | bld | 3.2^{a} | 2.8^{a} | bld |
| 5 | 33.1^{c} | bld | 19.7^{c} | 3.0^{a} | bld |
| 6 | 33.6^{c} | bld | 25.0^{c} | 3.4^{a} | bld |
| 7 | 38.2^{c} | 3.3^{a} | 80.8^{b} | 5.0^{a} | bld |
| 8 | 53.8^{c} | 16.7^{a} | 101.2^{c} | 14.0^{a} | bld |
| 9 | 57.4^{c} | 18.9^{a} | 114.6^{c} | 23.4^{b} | bld |
| 10 | 102.8^{c} | 24.6^{b} | 131.6^{c} | 64^{b} | bld |
| | | | | | |
| bld = below limit of detection | | | | | |
| ^{a} Negligible phenotype : slightly pale, slightly short | | | | | |
| ^{b} Mild phenotype : stunted by c. 1/3, leaves appear normal shape but | | | | | |
| ^{c} Strong phenotype : very stunted, deformed leaves, pale &/or mottled | | | | | |

### Example 3 - Transgenic plants comprising threonine insensitive AK:HSDH operably linked to a senescence specific promoter

*Nicotiana tabacum* plants, cultivar K326, were used to provide leaf discs which were co-cultivated with *Agrobacterium tumefasciens* which had been previously transformed (via electroporation) with a binary vector carrying the gene of interest (i.e. mutated AK:HSDH, the sequence of which is shown in Figure 7, i.e. SEQ ID No's:2, 3 and 4) under the control of the senescence-specitic promote, SAG 12 (the sequence of which is shown in Figure 6, i.e. SEQ ID No:1). A control population was simultaneously raised in which the binary vector contained the promoter but no AK:HSDH gene. These leaf discs were then processed through tissue culture protocols to provide plantlets (Horsch et al. Science 227: 1229-1231, 1985). Each plantlet resulted from a single transformation event wherein DNA was transferred from the bacterium and integrated into the plant's genomic DNA. These plants were transferred to the greenhouse and raised to maturity. Mature leaves were detached from the plants and placed in polythene bags in the dark at 35°C for 72h. After this time the leaf was used to provide leaf discs which were assessed for threonine content as described in Example 1 above.

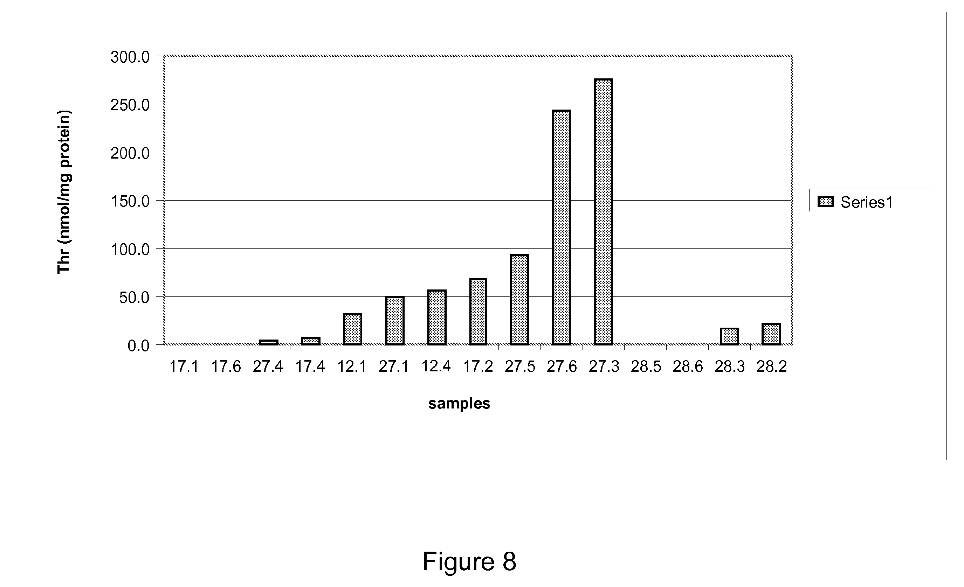

The results are shown in Figure 8 which shows that high levels of threonine were obtained in some plants. Advantageously, these plants grew normally. Therefore, these transgenic plants did not demonstrate a fitness loss, as observed with the plants described in Example 2.

### Example 4 - Field trials of transgenic plants comprising threonine insensitive AK:HSDH linked to the SAG12 promoter

Plant cell lines selected from the experimental population of SAGI2:Aspartate Kinase (pBNP 138-0253-001) were field grown during 2008 in North Carolina. The leaf was flue-cured and analysed for the presence of selected free amino acids. The analysis was by LC/MS, validated through internal and external standard calibration. Where the analyte falls above the range used for calibration it is indicated in the table as ">S" in Table 2, and, in the Figures, by a star over the relevant bar.

The sample list is divided into "AK" numbers, which are the K326 background lines modified with the genetic construct of the invention, and three representative controls; unmodified K326, unmodified KV1, unmodified NC71, which were all grown and cured at the same time under the same conditions.

**Table: 2**

| Sample | harvest | GLN | | ASN | | THR | | ASP | | HIS | | GLU | |
|---|---|---|---|---|---|---|---|---|---|---|---|---|---|
| | | Average | SD | Average | SD | Average | SD | Average | SD | Average | SD | Average | SD |
| AK1 | 1st | 76466 | 19305 | 139552 | 29565 | 34551 | 3380 | 38895 | 4454 | 26127 | 6508 | 26227 | 2130 |
| AK2 | 1st | 78798 | 14339 | 140286 | 21227 | 44034 | 7038 | 44448 | 3503 | 37819 | 7033 | 50543 | 9028 |
| AK3 | 1st | 109734 | 21911 | 150207 | 25335 | 42946 | 6047 | 50501 | 10027 | 32519 | 6922 | 44812 | 6544 |
| AK4 | 1st | 74311 | 12443 | 163639 | 30459 | 37780 | 6009 | 46438 | 5360 | 41722 | 7468 | 36233 | 2550 |
| AK5 | 1st | 106899 | 23166 | 153162 | 34012 | 35411 | 6485 | 58982 | 8213 | 33031 | 2304 | 50129 | 3586 |
| | | | | | | | | | | | | | |
| K326 | 1st | 56135 | 7320 | 118276 | 12377 | 11764 | 999 | 68531 | 24830 | 37632 | 3709 | 45471 | 2773 |
| KV1 | 1st | 85214 | 14761 | 162472 | 26365 | 22826 | 6668 | 53358 | 7400 | 35172 | 3280 | 38947 | 4572 |
| NC71 | 1st | 43057 | 3400 | 97084 | 7690 | 8372 | 895 | 36873 | 3794 | 25153 | 3036 | 43921 | 4622 |

| Sample | harvest | GLN | | ASN | | THR | | ASP | | HIS | | GLU | |
|---|---|---|---|---|---|---|---|---|---|---|---|---|---|
| | | Average | SD | Average | SD | Average | SD | Average | SD | Average | SD | Average | SD |
| AK1 | 2nd | 218157 | 27627 | >S | | 50958 | 4755 | 133901 | 11904 | 59546 | 8044 | 61724 | 8242 |
| AK2 | 2nd | 234444 | 34301 | >S | | 49778 | 7239 | 157169 | 27062 | 63921 | 9630 | 50071 | 5110 |
| AK3 | 2nd | 181130 | 32800 | 260874 | | 53067 | 4615 | 105212 | 24397 | 59104 | 18643 | 59861 | 9528 |
| AK4 | 2nd | 211848 | 24920 | 319595 | | 74562 | 15555 | 148343 | 38039 | 105304 | 23482 | 71246 | 7194 |
| AK5 | 2nd | 232277 | 12078 | >S | | 59158 | 14365 | 169550 | 16700 | 67586 | 7289 | 72360 | 6616 |
| | | | | | | | | | | | | | |
| K326 | 2nd | 217646 | 19769 | 240904 | 5139 | 32914 | 3997 | 119047 | 9574 | 46523 | 2805 | 64164 | 5334 |
| KV1 | 2nd | 198300 | 20990 | 261091 | 20159 | 29396 | 4423 | 108201 | 11556 | 43338 | 3238 | 61099 | 6799 |
| NC71 | 2nd | 190717 | 10617 | 263877 | 20229 | 31550 | 1907 | 120232 | 15665 | 46274 | 3637 | 53446 | 4406 |

| Sample | harvest | GLN | | ASN | | THR | | ASP | | HIS | | GLU | |
|---|---|---|---|---|---|---|---|---|---|---|---|---|---|
| | | Average | SD | Average | SD | Average | SD | Average | SD | Average | SD | Average | SD |
| AK1 | 3rd | 306597 | | >S | | 85347 | 17836 | 98494 | 21681 | 86954 | 15974 | 67067 | 14404 |
| AK2 | 3rd | 295089 | | >S | | 66725 | 9679 | 98979 | 14819 | 92280 | 6604 | 63414 | 8736 |
| AK3 | 3rd | 207486 | 25219 | 288603 | | 72014 | 13512 | 62618 | 8416 | 56082 | 12195 | 71410 | 16201 |
| AK4 | 3rd | >S | | >S | | 138831 | 30885 | 149836 | 19031 | 138219 | 23383 | 106872 | 14055 |
| AK5 | 3rd | >S | | >S | | 60075 | 9341 | 164408 | 28077 | 92613 | 12413 | 103457 | 10638 |
| | | | | | | | | | | | | | |
| K326 | 3rd | 240161 | 13666 | 292026 | #DIV/0! | 30765 | 3239 | 69660 | 6374 | 58525 | 8142 | 67404 | 5090 |
| KV1 | 3rd | 252047 | 20855 | 286494 | 14117 | 30812 | 4324 | 69793 | 8202 | 52625 | 5121 | 58637 | 6581 |
| NC71 | 3rd | 208263 | 24236 | 263515 | 13163 | 22598 | 2761 | 59525 | 8416 | 47625 | 3294 | 55226 | 4800 |

Referring to Figure 9, there is a shown a bar chart which illustrates the concentration of threonine produced by five test plants (AK1-AK5) compared to the three controls (K326, KV1 and NC7). As can be seen, all five of the test plants produced significantly more threonine than any of the controls. Furthermore, none of the five test plants suffered any cost to their growth fitness. These data strongly support the observation that test plants transformed with the construct according to the invention produce leaves with elevated (at least two to three-fold) free threonine under commercial field conditions, and that this persists though the curing process. Furthermore, the test plants were non-segregating lines that showed no yield penalties under these field conditions.

The inventors also assessed the concentration of several other amino acids (GLN: glutamine; GLU: glutamic acid; ASN: asparagine; ASP: aspartic acid; HIS: histidine) in the cured leaves produced from the field trial plants, and the data are shown in Figures 10-14.

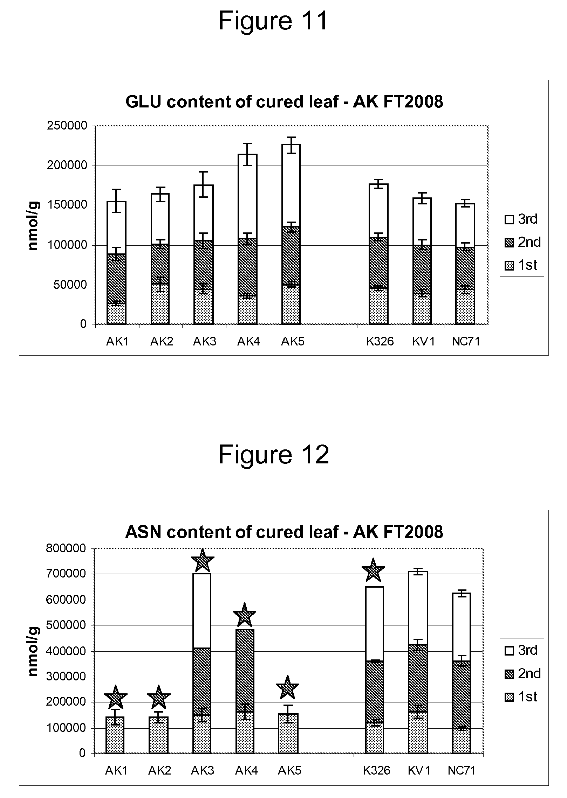

As can be seen in Figure 10, for cell line, AK3, the concentration of glutamine was comparable to that in the three control cell lines. As shown in Figure 11, AK1-3 had the same amount of glutamic acid as the controls, though AK4-5 had marginally higher concentrations. Figure 12 shows the respective concentrations of asparagine.

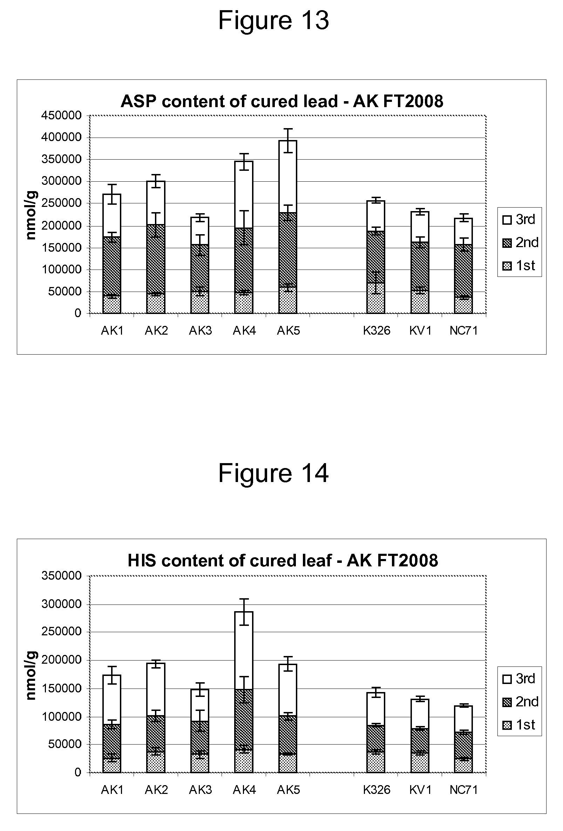

Referring to Figure 13, the concentrations of aspartic acid can be seen to be approximately the same in test cell lines AK1-AK3 as in the controls, although the concentrations appeared to be higher in AK4-5. Finally, as shown in Figure 14, the concentration of histidine was about the same thoughout, except that test cell line AK4 showed elevated levels.

In summary, Figures 10-14 clearly demonstrate that the concentrations of each of these amino acids were comparable or even higher than the controls, providing a good indication that the fitness of the transgenic plant had not been compromised.

## Claims

1. A genetic construct comprising a senescence specific promoter operably linked to a coding sequence encoding a polypeptide having threonine insensitive aspartate kinase activity.

2. A genetic construct according to claim 1, wherein the senescence specific promoter has been isolated from a senescence-associated gene in Arabidopsis.

3. A genetic construct according to either claim 1 or claim 2, wherein the senescence specific promoter is selected from a group consisting of SAG 12, SAG13, SAG101, SAG21 and SAG18.

4. A genetic construct according to any preceding claim, wherein the promoter comprises a nucleotide sequence substantially as set out in SEQ ID No. 1, or a functional variant or fragment thereof, wherein the variant or fragment thereof has at least 65% identity with SEQ ID No.1, and encodes a senescence specific promoter.

5. A genetic construct according to any preceding claim, wherein the polypeptide is a threonine insensitive aspartate kinase (AK) or a functional variant or fragment thereof wherein the variant or fragment thereof has at least 65% identity with SEQ ID NO: 5, SEQ ID NO: 6 or SEQ ID NO: 7, preferably a threonine insensitive bifunctional aspartate kinase-homoserine dehydrogenase (AK-HSDH) enzyme.

6. A genetic construct according to any preceding claim, wherein the coding sequence, which encodes the polypeptide having threonine insensitive aspartate kinase activity is derived from Arabidopsis spp., Zea spp., Flaveria spp., or Cleome spp..

7. A genetic construct according to either claim 5 or claim 6, wherein at least one threonine binding site of the AK-HSDH is mutated.

8. A genetic construct according to any one of claims 5-7, wherein the Arabidopsis AK-HSDH is mutated at Gln443 and/or Gln524, preferably mutated at Gln443 and Gln524.

9. A vector comprising the genetic construct according to any one of claims 1 to 8.

10. A method of producing a transgenic plant which accumulates a higher level of threonine in the leaves than a corresponding wild type plant cultured under the same conditions comprising:
i) transforming a plant cell with a genetic construct according to any one of claims 1 to 8, or the vector according to claim 9; and
ii) regenerating a plant from the transformed cell.

11. A cell comprising the genetic construct of any one of claims 1 to 8, or the vector according to claim 9.

12. A transgenic plant comprising the genetic construct of any one of claims 1 to 8, or the vector according to claim 9.

13. A transgenic plant according to claim 12, wherein the plant is from the Brassicaceae family, Poales family or the Solanaceae family, or wherein the plant is a tobacco plant.

14. A plant propagation product obtainable from the transgenic plant according to either claim 12 or claim 13, wherein the plant propagation product comprises the construct of any one of claims 1-8 or the vector of claim 9.

15. A plant propagation product according to claim 14, wherein the plant propagation product is a seed.

16. A harvested leaf containing a higher level of threonine than the corresponding level of threonine in a harvested leaf taken from a wild-type plant cultured under the same conditions, wherein the leaf comprises the construct according to any one of claims 1-8, or the vector according to claim 9.

17. A smoking article comprising threonine-enriched tobacco obtained from a mutant tobacco plant, which mutant is capable of over-producing threonine in senescent leaves wherein the mutant tobacco plant has been transformed with the genetic construct as claimed in any one of claims 1 to 8, or the vector according to claim 9.

18. A method of increasing the level of threonine in plant leaves above the corresponding wild type level without compromising plant fitness, comprising altering plant metabolism to achieve increased production of threonine after the initiation of leaf senescence, wherein the method comprises transforming the plant with the construct according to any one of claims 1 to 8, or the vector according to claim 9.

## Patentansprüche

1. Genkonstrukt, umfassend einen seneszenzspezifischen Promoter in operativer Verknüpfung mit einer Codiersequenz, die für ein Polypeptid mit der Aktivität einer threoninunempfindlichen Aspartatkinase codiert.

2. Genkonstrukt nach Anspruch 1, wobei der seneszenzspezifische Promoter aus einem seneszenzassoziierten Gen in Arabidopsis isoliert worden ist.

3. Genkonstrukt nach Anspruch 1 oder Anspruch 2, wobei der seneszenzspezifische Promoter aus einer Gruppe bestehend aus SAG12, SAG13, SAG101, SAG21 und SAG18 ausgewählt ist.

4. Genkonstrukt nach einem vorhergehenden Anspruch, wobei der Promoter eine Nukleotidsequenz wie im Wesentlichen in SEQ ID NO: 1 dargestellt oder eine funktionelle Variante oder ein funktionelles Fragment davon umfasst, wobei die Variante oder das Fragment davon mindestens 65% Identität zu SEQ ID NO: 1 aufweist und für einen seneszenzspezifischen Promoter codiert.

5. Genkonstrukt nach einem vorhergehenden Anspruch, wobei es sich bei dem Polypeptid um eine threoninunempfindliche Aspartatkinase (AK) oder eine funktionelle Variante oder ein funktionelles Fragment davon handelt, wobei die Variante oder das Fragment davon mindestens 65% Identität zu SEQ ID NO: 5, SEQ ID NO: 6 oder SEQ ID NO: 7 aufweist, vorzugsweise um ein threoninunempfindliches bifunktionelles Aspartatkinase-Homoserindehydrogenase (AK-HSDH)-Enzym.

6. Genkonstrukt nach einem vorhergehenden Anspruch, wobei die Codiersequenz, die für das Polypeptid mit Aktivität einer threoninunempfindlichen Aspartatkinase codiert, von Arabidopsis spp., Zea spp., Flaveria spp. oder Cleome spp. abgeleitet ist.

7. Genkonstrukt nach Anspruch 5 oder Anspruch 6, wobei mindestens eine Threoninbindungsstelle der AK-HSDH mutiert ist.

8. Genkonstrukt nach einem der Ansprüche 5-7, wobei die Arabidopsis-AK-HSDH an Gln443 und/oder Gln524 mutiert ist und vorzugsweise an Gln443 und Gln524 mutiert ist.

9. Vektor, umfassend das Genkonstrukt nach einem der Ansprüche 1 bis 8.

10. Verfahren zur Herstellung einer transgenen Pflanze, die einen höheren Threoningehalt in den Blättern akkumuliert als eine entsprechende Wildtyppflanze, die unter denselben Bedingungen kultiviert wird, das Folgendes umfasst:
i) Transformieren einer Pflanzenzelle mit einem Genkonstrukt nach einem der Ansprüche 1 bis 8 oder dem Vektor nach Anspruch 9; und
ii) Regenerieren einer Pflanze aus der transformierten Zelle.

11. Zelle, umfassend das Genkonstrukt nach einem der Ansprüche 1 bis 8 oder den Vektor nach Anspruch 9.

12. Transgene Pflanze, umfassend das Genkonstrukt nach einem der Ansprüche 1 bis 8 oder den Vektor nach Anspruch 9.

13. Transgene Pflanze nach Anspruch 12, wobei die Pflanze aus der Familie Brassicaceae, der Familie Poales oder der Familie Solanaceae stammt oder wobei es sich bei der Pflanze um eine Tabakpflanze handelt.

14. Pflanzliches Vermehrungsgut, erhältlich von der transgenen Pflanze nach Anspruch 12 oder Anspruch 13, wobei das pflanzliche Vermehrungsgut das Konstrukt nach einem der Ansprüche 1-8 oder den Vektor nach Anspruch 9 umfasst.

15. Pflanzliches Vermehrungsgut nach Anspruch 14, wobei es sich bei dem pflanzlichen Vermehrungsgut um einen Samen handelt.

16. Geerntetes Blatt, das einen höheren Threoningehalt als der entsprechende Threoningehalt in einem geernteten Blatt von einer Wildtyppflanze, die unter denselben Bedingungen kultiviert wird, aufweist, wobei das Blatt das Konstrukt nach einem der Ansprüche 1-8 oder den Vektor nach Anspruch 9 umfasst.

17. Rauchware, umfassend threoninangereicherten Tabak, der von einer Tabakpflanzenmutante erhalten wird, wobei die Mutante fähig ist, Threonin in alternden Blättern zu überproduzieren, wobei die Tabakpflanzenmutante mit dem Genkonstrukt nach einem der Ansprüche 1 bis 8 oder dem Vektor nach Anspruch 9 transformiert worden ist.

18. Verfahren zum Erhöhen des Threoningehalts in Pflanzenblättern auf über den entsprechenden Wildtypgehalt, ohne die Lebenskraft der Pflanze zu gefährden, umfassend das Verändern des Pflanzenmetabolismus, so dass nach dem Beginn der Blattseneszenz eine erhöhte Threoninproduktion erzielt wird, wobei das Verfahren das Transformieren der Pflanze mit dem Konstrukt nach einem der Ansprüche 1 bis 8 oder dem Vektor nach Anspruch 9 umfasst.

## Revendications

1. Construction génétique comprenant un promoteur spécifique de sénescence fonctionnellement lié à une séquence codante codant pour un polypeptide ayant une activité aspartate kinase insensible à la thréonine.

2. Construction génétique selon la revendication 1, dans laquelle le promoteur spécifique de sénescence a été isolé à partir d'un gène associé à la sénescence dans Arabidopsis.

3. Construction génétique selon la revendication 1 ou la revendication 2, dans laquelle le promoteur spécifique de sénescence est choisi dans un groupe constitué de SAG12, SAG13, SAG101, SAG21 et SAG18.

4. Construction génétique selon l'une quelconque des revendications précédentes, dans laquelle le promoteur comprend une séquence nucléotidique sensiblement telle que décrite dans SEQ ID NO: 1, ou un variant fonctionnel ou fragment de celle-ci, le variant ou fragment de celle-ci ayant au moins 65 % d'identité avec SEQ ID NO: 1, et codant pour un promoteur spécifique de sénescence.

5. Construction génétique selon l'une quelconque des revendications précédentes, dans laquelle le polypeptide est une aspartate kinase (AK) insensible à la thréonine ou un variant fonctionnel ou fragment de celle-ci, le variant ou fragment de celui-ci ayant au moins 65 % d'identité avec SEQ ID NO: 5, SEQ ID NO: 6 ou SEQ ID NO: 7, de préférence une enzyme aspartate kinase-homosérine déshydrogénase (AK-HSDH) bifonctionnelle insensible à la thréonine.

6. Construction génétique selon l'une quelconque des revendications précédentes, dans laquelle la séquence codante, qui code pour le polypeptide ayant une activité aspartate kinase insensible à la thréonine est dérivée d'Arabidopsis spp., Zea spp., Flaveria spp., ou Cleome spp.

7. Construction génétique selon la revendication 5 ou la revendication 6, dans laquelle au moins un site de liaison de thréonine de l'AK-HSDH est muté.

8. Construction génétique selon l'une quelconque des revendications 5 à 7, dans laquelle l'AK-HSDH d'Arabidopsis est mutée à Gln443 et/ou Gln524, de préférence mutée à Gln443 et Gln524.

9. Vecteur comprenant la construction génétique selon l'une quelconque des revendications 1 à 8.

10. Procédé de production d'une plante transgénique qui accumule un taux de thréonine dans les feuilles plus élevé qu'une plante de type sauvage correspondante cultivée dans les mêmes conditions comprenant :
i) la transformation d'une cellule de plante avec une construction génétique selon l'une quelconque des revendications 1 à 8, ou le vecteur selon la revendication 9 ; et
ii) la régénération d'une plante à partir de la cellule transformée.

11. Cellule comprenant la construction génétique de l'une quelconque des revendications 1 à 8, ou le vecteur selon la revendication 9.

12. Plante transgénique comprenant la construction génétique de l'une quelconque des revendications 1 à 8, ou le vecteur selon la revendication 9.

13. Plante transgénique selon la revendication 12, la plante étant de la famille Brassicaceae, la famille Poales ou la famille Solanaceae, ou la plante étant une plante de tabac.

14. Produit de propagation de plante pouvant être obtenu à partir de la plante transgénique selon la revendication 12 ou la revendication 13, le produit de propagation de plante comprenant la construction de l'une quelconque des revendications 1 à 8 ou le vecteur de la revendication 9.

15. Produit de propagation de plante selon la revendication 14, le produit de propagation de plante étant une graine.

16. Feuille récoltée contenant un taux de thréonine plus élevé que le taux de thréonine correspondant dans une feuille récoltée provenant d'une plante de type sauvage cultivée dans les mêmes conditions, la feuille comprenant la construction selon l'une quelconque des revendications 1 à 8, ou le vecteur selon la revendication 9.

17. Article pour fumeur comprenant du tabac enrichi en thréonine obtenu à partir d'une plante de tabac mutante, ledit mutant étant capable de surproduire de la thréonine dans des feuilles sénescentes, la plante de tabac mutante ayant été transformée avec la construction génétique selon l'une quelconque des revendications 1 à 8, ou le vecteur selon la revendication 9.

18. Procédé d'augmentation du taux de thréonine dans des feuilles de plante au-dessus du taux de type sauvage correspondant sans altérer la vigueur de la plante, comprenant la modification du métabolisme de la plante pour obtenir une production accrue de thréonine après l'initialisation de la sénescence des feuilles, le procédé comprenant la transformation de la plante avec la construction selon l'une quelconque des revendications 1 à 8, ou le vecteur selon la revendication 9.


### SEQUENCE LISTING

<110> Advanced Technologies (Cambridge) Limited
<120> Transgenic plants
<130> AXH/57177PCT1
<160> 13
<170> Patent In version 3.5
<210> 1
   <211> 2093
   <212> DNA
   <213> Arabidopsis thaliana
<400> 1
   tcgagacccg attgttattt ttagactgag acaaaaaagt agaatcgttg attgttaaaa      60
   tttaaaatta gtttcattac gtttcgataa aaaaatgatt agtttatcat agcttaatta     120
   tagcattgat ttctaaattt gttttttgac cacccttttt tctctctttg gtgttttctt     180
   aacattagaa gaacccataa caatgtacgt tcaaattaat taaaaacaat atttccaagt     240
   tttatatacg aaacttgttt tttttaatga aaacagttga atagttgatt atgaattagt     300
   tagatcaata ctcaatatat gatcaatgat gtatatatat gaactcagtt gttatacaag     360
   aaatgaaaat gctatttaaa tacagatcat gaagtgttaa aaagtgtcag aatatgacat     420
   gaagcgtttt gtcctaccgg gtattcgagt tataggtttg gatctctcaa gaatattttg     480
   ggccatacta gttatatttg ggcttaagcg ttttgcaaag agacgaggaa gaaagattgg     540
   gtcaagttaa caaaacagag acactcgtat tagttggtac tttggtagca agtcgattta     600
   tttgccagta aaaacttggt acacaactga caactcgtat cgttattagt ttgtacttgg     660
   tacctttggt tcaagaaaaa gttgatatag ttaaatcagt tgtgttcatg aggtgattgt     720
   gatttaattt gttgactagg gcgattcctt cacatcacaa taacaaagtt ttatagattt     780
   tttttttata acatttttgc cacgcttcgt aaagtttggt atttacaccg catttttccc     840
   tgtacaagaa ttcatatatt atttatttat atactccagt tgacaattat aagtttataa     900
   cgtttttaca attatttaaa taccatgtga agatccaaga atatgtctta cttcttcttt     960
   gtgtaagaaa actaactata tcactataat aaaataattc taatcattat atttgtaaat    1020
   atgcagttat ttgtcaattt tgaatttagt attttagacg ttatcacttc agccaaatat    1080
   gatttggatt taagtccaaa atgcaatttc gtacgtatcc ctcttgtcgt ctaatgatta    1140
   tttcaatatt tcttatatta tccctaacta cagagctaca tttatattgt attctaatga    1200
   cagggaaacc ttcatagaga ttcagataga tgaaattggt gggaaacatc attgaacagg    1260
   aaacttttag caaatcatat cgatttatct acaaaagaat acgtagcgta atgaagtcca    1320
   cttgttgtga atgactatga tttgatcaaa ttagttaatt ttgtcgaatc atttttcttt     1380
   ttgatttgat taagctttta acttgcacga atggttctct tgtgaataaa cagaatcttt     1440
   gaattcaaac tatttgatta gtgaaaagac aaaagaagat tccttgtttt tatgtgatta     1500
   gtgattttga tgcatgaaag gtacctacgt actacaagaa aaataaacat gtacgtaact     1560
   acgtatcagc atgtaaaagt atttttttcc aaataattta tactcatgat agattttttt     1620
   tttttgaaat gtcaattaaa aatgctttct taaatattaa ttttaattaa ttaaataagg     1680
   aaatatattt atgcaaaaca tcatcaacac atatccaact tcgaaaatct ctatagtaca     1740
   caagtagaga aattaaattt tactagatac aaacttccta atcatcaaat ataaatgttt     1800
   acaaaactaa ttaaacccac cactaaaatt aactaaaaat ccgagcaaag tgagtgaaca     1860
   agacttgatt tcaggttgat gtaggactaa aatgactacg tatcaaacat caacgatcat     1920
   ttagttatgt atgaatgaat gtagtcatta cttgtaaaac aaaaatgctt tgatttggat     1980
   caatcacttc atgtgaacat tagcaattac atcaacctta ttttcactat aaaaccccat     2040
   ctcagtaccc ttctgaagta atcaaattaa gagcaaaagt catttaactt agg            2093
<210> 2
   <211> 2751
   <212> DNA
   <213> Artificial
<220>
   <223> Aspartate kinase (At4g19710) single mutant (Q443A); R = A or G
<400> 2
   atggcgactc tgaagccgtc atttactgtt tctccgccga atagtaatcc gattagattt       60
   ggaagttttc cgccgcaatg ctttctccgt gttccgaaac cgcggcgact tatattgcct      120
   aggtttcgga agacgactgg tggtggcggc ggcttgattc gatgtgagct tccagatttt      180
   catctatcag caacagcaac tactgtatca ggtgtatcga cggtgaattt agtggatcaa      240
   gttcagattc ctaaaggtga aatgtggagt gttcacaagt ttggtgggac ttgtgtggga      300
   aactctcaga ggatcagaaa tgtagcagag gttataatca atgataattc cgaaagaaaa      360
   cttgtggttg tctcggcgat gtcgaaggtt acggacatga tgtatgactt aatccgcaag      420
   gcacaatcac gagatgattc ttatttatcc gcgttggaag ctgtcttgga aaagcatcgt      480
   ttaacagctc gtgaccttct cgatggagat gatctcgcta gtttcttgtc acatttgcat      540
   aatgatatta gtaatcttaa agcaatgctt cgtgctatat acatagctgg ccatgcatca      600
   gagtcgtttt cagattttgt tgcaggacat ggggagcttt ggtctgctca gatgctatca      660
   tatgttgtca gaaagactgg gcttgagtgc aagtggatgg atactagaga cgtgctcatt      720
   gttaatccca ccagctctaa tcaggttgat cctgattttg gtgaatctga gaagagactc      780
   gataaatggt tctccttaaa tccgtcgaaa attattattg cgactgggtt tattgctagc      840
   actccgcaaa atattccaac aactttgaaa agagatggga gtgatttctc agcagctatt      900
   atgggtgctt tattgagagc tcgtcaagta accatttgga cagatgttga tggtgtatac      960
   agtgcggatc ctcgtaaagt taatgaggca gtgatactcc agacactttc ttatcaagag     1020
   gcctgggaaa tgtcttattt tggagcaaat gtgttacatc ctcgcaccat cattcctgtg     1080
   atgcgatata atattccgat tgtgattaga aatattttca atctctctgc accgggaaca     1140
   ataatctgtc aacctcctga agatgattat gaccttaaac tgacaactcc tgtcaaaggg     1200
   tttgcaacta ttgacaattt ggccctcata aatgttgaag gtactggaat ggctggtgta     1260
   cccggtactg caagtgacat ttttggctgt gtaaaagatg ttggagctaa tgtgattatg     1320
   atatcagctg ctagcagtga gcattctgtg tgctttgctg tgcctgagaa ggaagtaaac     1380
   gcagtctctg aggcattgcg gtcgagattt agtgaagctt tacaagcggg acgtctttct     1440
   cagattgagg tgataccaaa ctgtagcatc ttagctgcag tcggccagaa aatggctagt     1500
   acacctggag ttagttgtac acttttcagt gctttggcga aggctaatat taatgtccga     1560
   gctatatctc arggttgttc tgagtacaat gttactgtcg ttattaaacg tgaagatagc     1620
   gttaaggcgt taagagctgt acactcgagg tttttcttgt caagaacaac attagcaatg     1680
   ggaatcgtag gaccgggctt gattggtgca acattacttg accagctgcg ggatcaggct     1740
   gctgttctca aacaagaatt taacattgat ctgcgtgttt tgggaatcac tggttcaaag     1800
   aagatgttat tgagtgacat tggtattgat ttgtcgagat ggagagaact tctaaacgag     1860
   aagggaacag aggcggattt ggataaattc actcaacaag tgcatggaaa tcattttatc     1920
   cccaactctg tagtggttga ttgtacagca gactctgcta ttgcaagccg ttactatgat     1980
   tggttacgaa agggaattca tgtcattacc ccaaataaaa aggctaactc aggtcccctc     2040
   gatcagtact tgaaactgag agatcttcaa aggaaatcct acactcatta cttctacgaa     2100
   gccactgttg gagctggtct tccaattatc agcactttac gtggtctcct tgagacagga     2160
   gataagatac tacgcataga gggcatttgc agtggaactt tgagttatct attcaacaat     2220
   tttgttggag atcgaagttt cagcgaggtt gtcactgaag caaagaacgc aggtttcact     2280
   gagcctgatc caagagatga tttatctgga actgatgttg caaggaaggt gattatcctc     2340
   gctcgagaat ctggactgaa attggacctc gctgatctcc ccattagaag tctcgtacca     2400
   gaacctctaa aaggatgcac ttctgttgaa gaattcatgg agaaactccc acagtacgat     2460
   ggagacctag caaaagaaag gctagatgct gaaaactctg gggaagttct gagatatgtt     2520
   ggagtggtgg acgctgttaa ccaaaaggga acagttgaac ttcgaagata caagaaagaa     2580
   catccatttg cgcagctcgc aggttcagac aacataatag ccttcacaac gacaaggtac 2640
   aaggatcatc cacttatagt ccgaggacct ggagctggtg ctcaagtcac ggccggtggt 2700
   atattcagcg acatactaag gcttgcatct tatctcggtg caccgtctta a 2751
<210> 3
   <211> 2751
   <212> DNA
   <213> Artificial
<220>
   <223> Aspartate kinase (At4g19710) single mutant (Q524A); R = A or G
<400> 3
   atggcgactc tgaagccgtc atttactgtt tctccgccga atagtaatcc gattagattt 60
   ggaagttttc cgccgcaatg ctttctccgt gttccgaaac cgcggcgact tatattgcct 120
   aggtttcgga agacgactgg tggtggcggc ggcttgattc gatgtgagct tccagatttt 180
   catctatcag caacagcaac tactgtatca ggtgtatcga cggtgaattt agtggatcaa 240
   gttcagattc ctaaaggtga aatgtggagt gttcacaagt ttggtgggac ttgtgtggga 300
   aactctcaga ggatcagaaa tgtagcagag gttataatca atgataattc cgaaagaaaa 360
   cttgtggttg tctcggcgat gtcgaaggtt acggacatga tgtatgactt aatccgcaag 420
   gcacaatcac gagatgattc ttatttatcc gcgttggaag ctgtcttgga aaagcatcgt 480
   ttaacagctc gtgaccttct cgatggagat gatctcgcta gtttcttgtc acatttgcat 540
   aatgatatta gtaatcttaa agcaatgctt cgtgctatat acatagctgg ccatgcatca 600
   gagtcgtttt cagattttgt tgcaggacat ggggagcttt ggtctgctca gatgctatca 660
   tatgttgtca gaaagactgg gcttgagtgc aagtggatgg atactagaga cgtgctcatt 720
   gttaatccca ccagctctaa tcaggttgat cctgattttg gtgaatctga gaagagactc 780
   gataaatggt tctccttaaa tccgtcgaaa attattattg cgactgggtt tattgctagc 840
   actccgcaaa atattccaac aactttgaaa agagatggga gtgatttctc agcagctatt 900
   atgggtgctt tattgagagc tcgtcaagta accatttgga cagatgttga tggtgtatac 960
   agtgcggatc ctcgtaaagt taatgaggca gtgatactcc agacactttc ttatcaagag 1020
   gcctgggaaa tgtcttattt tggagcaaat gtgttacatc ctcgcaccat cattcctgtg 1080
   atgcgatata atattccgat tgtgattaga aatattttca atctctctgc accgggaaca 1140
   ataatctgtc aacctcctga agatgattat gaccttaaac tgacaactcc tgtcaaaggg 1200
   tttgcaacta ttgacaattt ggccctcata aatgttgaag gtactggaat ggctggtgta 1260
   cccggtactg caagtgacat ttttggctgt gtaaaagatg ttggagctaa tgtgattatg 1320
   atatcacarg ctagcagtga gcattctgtg tgctttgctg tgcctgagaa ggaagtaaac 1380
   gcagtctctg aggcattgcg gtcgagattt agtgaagctt tacaagcggg acgtctttct 1440
   cagattgagg tgataccaaa ctgtagcatc ttagctgcag tcggccagaa aatggctagt 1500
   acacctggag ttagttgtac acttttcagt gctttggcga aggctaatat taatgtccga 1560
   gctatatctg ctggttgttc tgagtacaat gttactgtcg ttattaaacg tgaagatagc 1620
   gttaaggcgt taagagctgt acactcgagg tttttcttgt caagaacaac attagcaatg 1680
   ggaatcgtag gaccgggctt gattggtgca acattacttg accagctgcg ggatcaggct 1740
   gctgttctca aacaagaatt taacattgat ctgcgtgttt tgggaatcac tggttcaaag 1800
   aagatgttat tgagtgacat tggtattgat ttgtcgagat ggagagaact tctaaacgag 1860
   aagggaacag aggcggattt ggataaattc actcaacaag tgcatggaaa tcattttatc 1920
   cccaactctg tagtggttga ttgtacagca gactctgcta ttgcaagccg ttactatgat 1980
   tggttacgaa agggaattca tgtcattacc ccaaataaaa aggctaactc aggtcccctc 2040
   gatcagtact tgaaactgag agatcttcaa aggaaatcct acactcatta cttctacgaa 2100
   gccactgttg gagctggtct tccaattatc agcactttac gtggtctcct tgagacagga 2160
   gataagatac tacgcataga gggcatttgc agtggaactt tgagttatct attcaacaat 2220
   tttgttggag atcgaagttt cagcgaggtt gtcactgaag caaagaacgc aggtttcact 2280
   gagcctgatc caagagatga tttatctgga actgatgttg caaggaaggt gattatcctc 2340
   gctcgagaat ctggactgaa attggacctc gctgatctcc ccattagaag tctcgtacca 2400
   gaacctctaa aaggatgcac ttctgttgaa gaattcatgg agaaactccc acagtacgat 2460
   ggagacctag caaaagaaag gctagatgct gaaaactctg gggaagttct gagatatgtt 2520
   ggagtggtgg acgctgttaa ccaaaaggga acagttgaac ttcgaagata caagaaagaa 2580
   catccatttg cgcagctcgc aggttcagac aacataatag ccttcacaac gacaaggtac 2640
   aaggatcatc cacttatagt ccgaggacct ggagctggtg ctcaagtcac ggccggtggt 2700
   atattcagcg acatactaag gcttgcatct tatctcggtg caccgtctta a 2751
<210> 4
   <211> 2751
   <212> DNA
   <213> Artificial
<220>
   <223> Aspartate kinase (At4g19710) double mutant (Q443A; Q524A); R = A or G
<400> 4
   atggcgactc tgaagccgtc atttactgtt tctccgccga atagtaatcc gattagattt 60
   ggaagttttc cgccgcaatg ctttctccgt gttccgaaac cgcggcgact tatattgcct      120
   aggtttcgga agacgactgg tggtggcggc ggcttgattc gatgtgagct tccagatttt      180
   catctatcag caacagcaac tactgtatca ggtgtatcga cggtgaattt agtggatcaa      240
   gttcagattc ctaaaggtga aatgtggagt gttcacaagt ttggtgggac ttgtgtggga      300
   aactctcaga ggatcagaaa tgtagcagag gttataatca atgataattc cgaaagaaaa      360
   cttgtggttg tctcggcgat gtcgaaggtt acggacatga tgtatgactt aatccgcaag      420
   gcacaatcac gagatgattc ttatttatcc gcgttggaag ctgtcttgga aaagcatcgt      480
   ttaacagctc gtgaccttct cgatggagat gatctcgcta gtttcttgtc acatttgcat      540
   aatgatatta gtaatcttaa agcaatgctt cgtgctatat acatagctgg ccatgcatca      600
   gagtcgtttt cagattttgt tgcaggacat ggggagcttt ggtctgctca gatgctatca      660
   tatgttgtca gaaagactgg gcttgagtgc aagtggatgg atactagaga cgtgctcatt      720
   gttaatccca ccagctctaa tcaggttgat cctgattttg gtgaatctga gaagagactc      780
   gataaatggt tctccttaaa tccgtcgaaa attattattg cgactgggtt tattgctagc      840
   actccgcaaa atattccaac aactttgaaa agagatggga gtgatttctc agcagctatt      900
   atgggtgctt tattgagagc tcgtcaagta accatttgga cagatgttga tggtgtatac      960
   agtgcggatc ctcgtaaagt taatgaggca gtgatactcc agacactttc ttatcaagag     1020
   gcctgggaaa tgtcttattt tggagcaaat gtgttacatc ctcgcaccat cattcctgtg     1080
   atgcgatata atattccgat tgtgattaga aatattttca atctctctgc accgggaaca     1140
   ataatctgtc aacctcctga agatgattat gaccttaaac tgacaactcc tgtcaaaggg     1200
   tttgcaacta ttgacaattt ggccctcata aatgttgaag gtactggaat ggctggtgta     1260
   cccggtactg caagtgacat ttttggctgt gtaaaagatg ttggagctaa tgtgattatg     1320
   atatcagctg ctagcagtga gcattctgtg tgctttgctg tgcctgagaa ggaagtaaac     1380
   gcagtctctg aggcattgcg gtcgagattt agtgaagctt tacaagcggg acgtctttct     1440
   cagattgagg tgataccaaa ctgtagcatc ttagctgcag tcggccagaa aatggctagt     1500
   acacctggag ttagttgtac acttttcagt gctttggcga aggctaatat taatgtccga     1560
   gctatatctg ctggttgttc tgagtacaat gttactgtcg ttattaaacg tgaagatagc     1620
   gttaaggcgt taagagctgt acactcgagg tttttcttgt caagaacaac attagcaatg     1680
   ggaatcgtag gaccgggctt gattggtgca acattacttg accagctgcg ggatcaggct     1740
   gctgttctca aacaagaatt taacattgat ctgcgtgttt tgggaatcac tggttcaaag     1800
   aagatgttat tgagtgacat tggtattgat ttgtcgagat ggagagaact tctaaacgag     1860
   aagggaacag aggcggattt ggataaattc actcaacaag tgcatggaaa tcattttatc     1920
   cccaactctg tagtggttga ttgtacagca gactctgcta ttgcaagccg ttactatgat     1980
   tggttacgaa agggaattca tgtcattacc ccaaataaaa aggctaactc aggtcccctc     2040
   gatcagtact tgaaactgag agatcttcaa aggaaatcct acactcatta cttctacgaa     2100
   gccactgttg gagctggtct tccaattatc agcactttac gtggtctcct tgagacagga     2160
   gataagatac tacgcataga gggcatttgc agtggaactt tgagttatct attcaacaat     2220
   tttgttggag atcgaagttt cagcgaggtt gtcactgaag caaagaacgc aggtttcact     2280
   gagcctgatc caagagatga tttatctgga actgatgttg caaggaaggt gattatcctc     2340
   gctcgagaat ctggactgaa attggacctc gctgatctcc ccattagaag tctcgtacca     2400
   gaacctctaa aaggatgcac ttctgttgaa gaattcatgg agaaactccc acagtacgat     2460
   ggagacctag caaaagaaag gctagatgct gaaaactctg gggaagttct gagatatgtt     2520
   ggagtggtgg acgctgttaa ccaaaaggga acagttgaac ttcgaagata caagaaagaa     2580
   catccatttg cgcagctcgc aggttcagac aacataatag ccttcacaac gacaaggtac     2640
   aaggatcatc cacttatagt ccgaggacct ggagctggtg ctcaagtcac ggccggtggt     2700
   atattcagcg acatactaag gcttgcatct tatctcggtg caccgtctta a              2751
<210> 5
   <211> 916
   <212> PRT
   <213> Artificial
<220>
   <223> Aspartate kinase (At4g19710) single mutant (Q443A)
<400> 5
   Met Ala Thr Leu Lys Pro Ser Phe Thr Val Ser Pro Pro Asn Ser Asn
   1               5                   10                  15
   Pro Ile Arg Phe Gly Ser Phe Pro Pro Gln Cys Phe Leu Arg Val Pro
               20                  25                  30
   Lys Pro Arg Arg Leu Ile Leu Pro Arg Phe Arg Lys Thr Thr Gly Gly
           35                  40                  45
   Gly Gly Gly Leu Ile Arg Cys Glu Leu Pro Asp Phe His Leu Ser Ala
       50                  55                  60
   Thr Ala Thr Thr Val Ser Gly Val Ser Thr Val Asn Leu Val Asp Gln
   65                  70                  75                  80
   Val Gln Ile Pro Lys Gly Glu Met Trp Ser Val His Lys Phe Gly Gly
                   85                  90                  95
   Thr Cys Val Gly Asn Ser Gln Arg Ile Arg Asn Val Ala Glu Val Ile
               100                 105                 110
   Ile Asn Asp Asn Ser Glu Arg Lys Leu Val Val Val Ser Ala Met Ser
           115                 120                 125
   Lys Val Thr Asp Met Met Tyr Asp Leu Ile Arg Lys Ala Gln Ser Arg
       130                 135                 140
   Asp Asp Ser Tyr Leu Ser Ala Leu Glu Ala Val Leu Glu Lys His Arg
   145                 150                 155                 160
   Leu Thr Ala Arg Asp Leu Leu Asp Gly Asp Asp Leu Ala Ser Phe Leu
                   165                 170                 175
   Ser His Leu His Asn Asp Ile Ser Asn Leu Lys Ala Met Leu Arg Ala
               180                 185                 190
   Ile Tyr Ile Ala Gly His Ala Ser Glu Ser Phe Ser Asp Phe Val Ala
           195                 200                 205
   Gly His Gly Glu Leu Trp Ser Ala Gln Met Leu Ser Tyr Val Val Arg
       210                 215                 220
   Lys Thr Gly Leu Glu Cys Lys Trp Met Asp Thr Arg Asp Val Leu Ile
   225                 230                 235                 240
   Val Asn Pro Thr Ser Ser Asn Gln Val Asp Pro Asp Phe Gly Glu Ser
                   245                 250                 255
   Glu Lys Arg Leu Asp Lys Trp Phe Ser Leu Asn Pro Ser Lys Ile Ile
               260                 265                 270
   Ile Ala Thr Gly Phe Ile Ala Ser Thr Pro Gln Asn Ile Pro Thr Thr
           275                 280                 285
   Leu Lys Arg Asp Gly Ser Asp Phe Ser Ala Ala Ile Met Gly Ala Leu
       290                 295                 300
   Leu Arg Ala Arg Gln Val Thr Ile Trp Thr Asp Val Asp Gly Val Tyr
   305                 310                 315                 320
   Ser Ala Asp Pro Arg Lys Val Asn Glu Ala Val Ile Leu Gln Thr Leu
                   325                 330                 335
   Ser Tyr Gln Glu Ala Trp Glu Met Ser Tyr Phe Gly Ala Asn Val Leu
               340                 345                 350
   His Pro Arg Thr Ile Ile Pro Val Met Arg Tyr Asn Ile Pro Ile Val
           355                 360                 365
   Ile Arg Asn Ile Phe Asn Leu Ser Ala Pro Gly Thr Ile Ile Cys Gln
       370                 375                 380
   Pro Pro Glu Asp Asp Tyr Asp Leu Lys Leu Thr Thr Pro Val Lys Gly
   385                 390                 395                 400
   Phe Ala Thr Ile Asp Asn Leu Ala Leu Ile Asn Val Glu Gly Thr Gly
                   405                 410                 415
   Met Ala Gly Val Pro Gly Thr Ala Ser Asp Ile Phe Gly Cys Val Lys
               420                 425                 430
   Asp Val Gly Ala Asn Val Ile Met Ile Ser Ala Ala Ser Ser Glu His
           435                 440                 445
   Ser Val Cys Phe Ala Val Pro Glu Lys Glu Val Asn Ala Val Ser Glu
       450                 455                 460
   Ala Leu Arg Ser Arg Phe Ser Glu Ala Leu Gln Ala Gly Arg Leu Ser
   465                 470                 475                 480
   Gln Ile Glu Val Ile Pro Asn Cys Ser Ile Leu Ala Ala Val Gly Gln
                   485                 490                 495
   Lys Met Ala Ser Thr Pro Gly Val Ser Cys Thr Leu Phe Ser Ala Leu
               500                 505                 510
   Ala Lys Ala Asn Ile Asn Val Arg Ala Ile Ser Gln Gly Cys Ser Glu
           515                 520                 525
   Tyr Asn Val Thr Val Val Ile Lys Arg Glu Asp Ser Val Lys Ala Leu
       530                 535                 540
   Arg Ala Val His Ser Arg Phe Phe Leu Ser Arg Thr Thr Leu Ala Met
   545                 550                 555                 560
   Gly Ile Val Gly Pro Gly Leu Ile Gly Ala Thr Leu Leu Asp Gln Leu
                   565                 570                 575
   Arg Asp Gln Ala Ala Val Leu Lys Gln Glu Phe Asn Ile Asp Leu Arg
               580                 585                 590
   Val Leu Gly Ile Thr Gly Ser Lys Lys Met Leu Leu Ser Asp Ile Gly
           595                 600                 605
   Ile Asp Leu Ser Arg Trp Arg Glu Leu Leu Asn Glu Lys Gly Thr Glu
       610                 615                 620
   Ala Asp Leu Asp Lys Phe Thr Gln Gln Val His Gly Asn His Phe Ile
   625                 630                 635                 640
   Pro Asn Ser Val Val Val Asp Cys Thr Ala Asp Ser Ala Ile Ala Ser
                   645                 650                 655
   Arg Tyr Tyr Asp Trp Leu Arg Lys Gly Ile His Val Ile Thr Pro Asn
               660                 665                 670
   Lys Lys Ala Asn Ser Gly Pro Leu Asp Gln Tyr Leu Lys Leu Arg Asp
           675                 680                 685
   Leu Gln Arg Lys Ser Tyr Thr His Tyr Phe Tyr Glu Ala Thr Val Gly
       690                 695                 700
   Ala Gly Leu Pro Ile Ile Ser Thr Leu Arg Gly Leu Leu Glu Thr Gly
   705                 710                 715                 720
   Asp Lys Ile Leu Arg Ile Glu Gly Ile Cys Ser Gly Thr Leu Ser Tyr
                   725                 730                 735
   Leu Phe Asn Asn Phe Val Gly Asp Arg Ser Phe Ser Glu Val Val Thr
               740                 745                 750
   Glu Ala Lys Asn Ala Gly Phe Thr Glu Pro Asp Pro Arg Asp Asp Leu
           755                 760                 765
   Ser Gly Thr Asp Val Ala Arg Lys Val Ile Ile Leu Ala Arg Glu Ser
       770                 775                 780
   Gly Leu Lys Leu Asp Leu Ala Asp Leu Pro Ile Arg Ser Leu Val Pro
   785                 790                 795                 800
   Glu Pro Leu Lys Gly Cys Thr Ser Val Glu Glu Phe Met Glu Lys Leu
                   805                 810                 815
   Pro Gln Tyr Asp Gly Asp Leu Ala Lys Glu Arg Leu Asp Ala Glu Asn
               820                 825                 830
   Ser Gly Glu Val Leu Arg Tyr Val Gly Val Val Asp Ala Val Asn Gln
           835                 840                 845
   Lys Gly Thr Val Glu Leu Arg Arg Tyr Lys Lys Glu His Pro Phe Ala
       850                 855                 860
   Gln Leu Ala Gly Ser Asp Asn Ile Ile Ala Phe Thr Thr Thr Arg Tyr
   865                 870                 875                 880
   Lys Asp His Pro Leu Ile Val Arg Gly Pro Gly Ala Gly Ala Gln Val
                   885                 890                 895
   Thr Ala Gly Gly Ile Phe Ser Asp Ile Leu Arg Leu Ala Ser Tyr Leu
               900                 905                 910
   Gly Ala Pro Ser
           915
<210> 6
   <211> 916
   <212> PRT
   <213> Artificial
<220>
   <223> Aspartate kinase (At4g19710) single mutant (Q524A)
<400> 6
   Met Ala Thr Leu Lys Pro Ser Phe Thr Val Ser Pro Pro Asn Ser Asn
   1               5                   10                  15
   Pro Ile Arg Phe Gly Ser Phe Pro Pro Gln Cys Phe Leu Arg Val Pro
               20                  25                  30
   Lys Pro Arg Arg Leu Ile Leu Pro Arg Phe Arg Lys Thr Thr Gly Gly
           35                  40                  45
   Gly Gly Gly Leu Ile Arg Cys Glu Leu Pro Asp Phe His Leu Ser Ala
       50                  55                  60
   Thr Ala Thr Thr Val Ser Gly Val Ser Thr Val Asn Leu Val Asp Gln
   65                  70                  75                  80
   Val Gln Ile Pro Lys Gly Glu Met Trp Ser Val His Lys Phe Gly Gly
                   85                  90                  95
   Thr Cys Val Gly Asn Ser Gln Arg Ile Arg Asn Val Ala Glu Val Ile
               100                 105                 110
   Ile Asn Asp Asn Ser Glu Arg Lys Leu Val Val Val Ser Ala Met Ser
           115                 120                 125
   Lys Val Thr Asp Met Met Tyr Asp Leu Ile Arg Lys Ala Gln Ser Arg
       130                 135                 140
   Asp Asp Ser Tyr Leu Ser Ala Leu Glu Ala Val Leu Glu Lys His Arg
   145                 150                 155                 160
   Leu Thr Ala Arg Asp Leu Leu Asp Gly Asp Asp Leu Ala Ser Phe Leu
                   165                 170                 175
   Ser His Leu His Asn Asp Ile Ser Asn Leu Lys Ala Met Leu Arg Ala
               180                 185                 190
   Ile Tyr Ile Ala Gly His Ala Ser Glu Ser Phe Ser Asp Phe Val Ala
           195                 200                 205
   Gly His Gly Glu Leu Trp Ser Ala Gln Met Leu Ser Tyr Val Val Arg
       210                 215                 220
   Lys Thr Gly Leu Glu Cys Lys Trp Met Asp Thr Arg Asp Val Leu Ile
   225                 230                 235                 240
   Val Asn Pro Thr Ser Ser Asn Gln Val Asp Pro Asp Phe Gly Glu Ser
                   245                 250                 255
   Glu Lys Arg Leu Asp Lys Trp Phe Ser Leu Asn Pro Ser Lys Ile Ile
               260                 265                 270
   Ile Ala Thr Gly Phe Ile Ala Ser Thr Pro Gln Asn Ile Pro Thr Thr
           275                 280                 285
   Leu Lys Arg Asp Gly Ser Asp Phe Ser Ala Ala Ile Met Gly Ala Leu
       290                 295                 300
   Leu Arg Ala Arg Gln Val Thr Ile Trp Thr Asp Val Asp Gly Val Tyr
   305                 310                 315                 320
   Ser Ala Asp Pro Arg Lys Val Asn Glu Ala Val Ile Leu Gln Thr Leu
                   325                 330                 335
   Ser Tyr Gln Glu Ala Trp Glu Met Ser Tyr Phe Gly Ala Asn Val Leu
   340 345 350
   His Pro Arg Thr Ile Ile Pro Val Met Arg Tyr Asn Ile Pro Ile Val
   355 360 365
   Ile Arg Asn Ile Phe Asn Leu Ser Ala Pro Gly Thr Ile Ile Cys Gln
   370 375 380
   Pro Pro Glu Asp Asp Tyr Asp Leu Lys Leu Thr Thr Pro Val Lys Gly
   385 390 395 400
   Phe Ala Thr Ile Asp Asn Leu Ala Leu Ile Asn Val Glu Gly Thr Gly
   405 410 415
   Met Ala Gly Val Pro Gly Thr Ala Ser Asp Ile Phe Gly Cys Val Lys
   420 425 430
   Asp Val Gly Ala Asn Val Ile Met Ile Ser Gln Ala Ser Ser Glu His
   435 440 445
   Ser Val Cys Phe Ala Val Pro Glu Lys Glu Val Asn Ala Val Ser Glu
   450 455 460
   Ala Leu Arg Ser Arg Phe Ser Glu Ala Leu Gln Ala Gly Arg Leu Ser
   465 470 475 480
   Gln Ile Glu Val Ile Pro Asn Cys Ser Ile Leu Ala Ala Val Gly Gln
   485 490 495
   Lys Met Ala Ser Thr Pro Gly Val Ser Cys Thr Leu Phe Ser Ala Leu
   500 505 510
   Ala Lys Ala Asn Ile Asn Val Arg Ala Ile Ser Ala Gly Cys Ser Glu
   515 520 525
   Tyr Asn Val Thr Val Val Ile Lys Arg Glu Asp Ser Val Lys Ala Leu
   530 535 540
   Arg Ala Val His Ser Arg Phe Phe Leu Ser Arg Thr Thr Leu Ala Met
   545 550 555 560
   Gly Ile Val Gly Pro Gly Leu Ile Gly Ala Thr Leu Leu Asp Gln Leu
   565 570 575
   Arg Asp Gln Ala Ala Val Leu Lys Gln Glu Phe Asn Ile Asp Leu Arg
               580                 585                 590
   Val Leu Gly Ile Thr Gly Ser Lys Lys Met Leu Leu Ser Asp Ile Gly
           595                 600                 605
   Ile Asp Leu Ser Arg Trp Arg Glu Leu Leu Asn Glu Lys Gly Thr Glu
       610                 615                 620
   Ala Asp Leu Asp Lys Phe Thr Gln Gln Val His Gly Asn His Phe Ile
   625                 630                 635                 640
   Pro Asn Ser Val Val Val Asp Cys Thr Ala Asp Ser Ala Ile Ala Ser
                   645                 650                 655
   Arg Tyr Tyr Asp Trp Leu Arg Lys Gly Ile His Val Ile Thr Pro Asn
               660                 665                 670
   Lys Lys Ala Asn Ser Gly Pro Leu Asp Gln Tyr Leu Lys Leu Arg Asp
           675                 680                 685
   Leu Gln Arg Lys Ser Tyr Thr His Tyr Phe Tyr Glu Ala Thr Val Gly
       690                 695                 700
   Ala Gly Leu Pro Ile Ile Ser Thr Leu Arg Gly Leu Leu Glu Thr Gly
   705                 710                 715                 720
   Asp Lys Ile Leu Arg Ile Glu Gly Ile Cys Ser Gly Thr Leu Ser Tyr
                   725                 730                 735
   Leu Phe Asn Asn Phe Val Gly Asp Arg Ser Phe Ser Glu Val Val Thr
               740                 745                 750
   Glu Ala Lys Asn Ala Gly Phe Thr Glu Pro Asp Pro Arg Asp Asp Leu
           755                 760                 765
   Ser Gly Thr Asp Val Ala Arg Lys Val Ile Ile Leu Ala Arg Glu Ser
       770                 775                 780
   Gly Leu Lys Leu Asp Leu Ala Asp Leu Pro Ile Arg Ser Leu Val Pro
   785                 790                 795                 800
   Glu Pro Leu Lys Gly Cys Thr Ser Val Glu Glu Phe Met Glu Lys Leu
                   805                 810                 815
   Pro Gln Tyr Asp Gly Asp Leu Ala Lys Glu Arg Leu Asp Ala Glu Asn
               820                 825                 830
   Ser Gly Glu Val Leu Arg Tyr Val Gly Val Val Asp Ala Val Asn Gln
           835                 840                 845
   Lys Gly Thr Val Glu Leu Arg Arg Tyr Lys Lys Glu His Pro Phe Ala
       850                 855                 860
   Gln Leu Ala Gly Ser Asp Asn Ile Ile Ala Phe Thr Thr Thr Arg Tyr
   865                 870                 875                 880
   Lys Asp His Pro Leu Ile Val Arg Gly Pro Gly Ala Gly Ala Gln Val
                   885                 890                 895
   Thr Ala Gly Gly Ile Phe Ser Asp Ile Leu Arg Leu Ala Ser Tyr Leu
               900                 905                 910
   Gly Ala Pro Ser
           915
<210> 7
   <211> 916
   <212> PRT
   <213> Artificial
<220>
   <223> Aspartate kinase (At4g19710) double mutant (Q443A; Q524A)
<400> 7
   Met Ala Thr Leu Lys Pro Ser Phe Thr Val Ser Pro Pro Asn Ser Asn
   1               5                   10                  15
   Pro Ile Arg Phe Gly Ser Phe Pro Pro Gln Cys Phe Leu Arg Val Pro
               20                  25                  30
   Lys Pro Arg Arg Leu Ile Leu Pro Arg Phe Arg Lys Thr Thr Gly Gly
           35                  40                  45
   Gly Gly Gly Leu Ile Arg Cys Glu Leu Pro Asp Phe His Leu Ser Ala
       50                  55                  60
   Thr Ala Thr Thr Val Ser Gly Val Ser Thr Val Asn Leu Val Asp Gln
   65                  70                  75                  80
   Val Gln Ile Pro Lys Gly Glu Met Trp Ser Val His Lys Phe Gly Gly
                   85                  90                  95
   Thr Cys Val Gly Asn Ser Gln Arg Ile Arg Asn Val Ala Glu Val Ile
               100                 105                 110
   Ile Asn Asp Asn Ser Glu Arg Lys Leu Val Val Val Ser Ala Met Ser
           115                 120                 125
   Lys Val Thr Asp Met Met Tyr Asp Leu Ile Arg Lys Ala Gln Ser Arg
       130                 135                 140
   Asp Asp Ser Tyr Leu Ser Ala Leu Glu Ala Val Leu Glu Lys His Arg
   145                 150                 155                 160
   Leu Thr Ala Arg Asp Leu Leu Asp Gly Asp Asp Leu Ala Ser Phe Leu
                   165                 170                 175
   Ser His Leu His Asn Asp Ile Ser Asn Leu Lys Ala Met Leu Arg Ala
               180                 185                 190
   Ile Tyr Ile Ala Gly His Ala Ser Glu Ser Phe Ser Asp Phe Val Ala
           195                 200                 205
   Gly His Gly Glu Leu Trp Ser Ala Gln Met Leu Ser Tyr Val Val Arg
       210                 215                 220
   Lys Thr Gly Leu Glu Cys Lys Trp Met Asp Thr Arg Asp Val Leu Ile
   225                 230                 235                 240
   Val Asn Pro Thr Ser Ser Asn Gln Val Asp Pro Asp Phe Gly Glu Ser
                   245                 250                 255
   Glu Lys Arg Leu Asp Lys Trp Phe Ser Leu Asn Pro Ser Lys Ile Ile
               260                 265                 270
   Ile Ala Thr Gly Phe Ile Ala Ser Thr Pro Gln Asn Ile Pro Thr Thr
           275                 280                 285
   Leu Lys Arg Asp Gly Ser Asp Phe Ser Ala Ala Ile Met Gly Ala Leu
       290                 295                 300
   Leu Arg Ala Arg Gln Val Thr Ile Trp Thr Asp Val Asp Gly Val Tyr
   305                 310                 315                 320
   Ser Ala Asp Pro Arg Lys Val Asn Glu Ala Val Ile Leu Gln Thr Leu
                   325                 330                 335
   Ser Tyr Gln Glu Ala Trp Glu Met Ser Tyr Phe Gly Ala Asn Val Leu
               340                 345                 350
   His Pro Arg Thr Ile Ile Pro Val Met Arg Tyr Asn Ile Pro Ile Val
           355                 360                 365
   Ile Arg Asn Ile Phe Asn Leu Ser Ala Pro Gly Thr Ile Ile Cys Gln
       370                 375                 380
   Pro Pro Glu Asp Asp Tyr Asp Leu Lys Leu Thr Thr Pro Val Lys Gly
   385                 390                 395                 400
   Phe Ala Thr Ile Asp Asn Leu Ala Leu Ile Asn Val Glu Gly Thr Gly
                   405                 410                 415
   Met Ala Gly Val Pro Gly Thr Ala Ser Asp Ile Phe Gly Cys Val Lys
               420                 425                 430
   Asp Val Gly Ala Asn Val Ile Met Ile Ser Ala Ala Ser Ser Glu His
           435                 440                 445
   Ser Val Cys Phe Ala Val Pro Glu Lys Glu Val Asn Ala Val Ser Glu
       450                 455                 460
   Ala Leu Arg Ser Arg Phe Ser Glu Ala Leu Gln Ala Gly Arg Leu Ser
   465                 470                 475                 480
   Gln Ile Glu Val Ile Pro Asn Cys Ser Ile Leu Ala Ala Val Gly Gln
                   485                 490                 495
   Lys Met Ala Ser Thr Pro Gly Val Ser Cys Thr Leu Phe Ser Ala Leu
               500                 505                 510
   Ala Lys Ala Asn Ile Asn Val Arg Ala Ile Ser Ala Gly Cys Ser Glu
           515                 520                 525
   Tyr Asn Val Thr Val Val Ile Lys Arg Glu Asp Ser Val Lys Ala Leu
       530                 535                 540
   Arg Ala Val His Ser Arg Phe Phe Leu Ser Arg Thr Thr Leu Ala Met
   545                 550                 555                 560
   Gly Ile Val Gly Pro Gly Leu Ile Gly Ala Thr Leu Leu Asp Gln Leu
                   565                 570                 575
   Arg Asp Gln Ala Ala Val Leu Lys Gln Glu Phe Asn Ile Asp Leu Arg
               580                 585                 590
   Val Leu Gly Ile Thr Gly Ser Lys Lys Met Leu Leu Ser Asp Ile Gly
           595                 600                 605
   Ile Asp Leu Ser Arg Trp Arg Glu Leu Leu Asn Glu Lys Gly Thr Glu
       610                 615                 620
   Ala Asp Leu Asp Lys Phe Thr Gln Gln Val His Gly Asn His Phe Ile
   625                 630                 635                 640
   Pro Asn Ser Val Val Val Asp Cys Thr Ala Asp Ser Ala Ile Ala Ser
                   645                 650                 655
   Arg Tyr Tyr Asp Trp Leu Arg Lys Gly Ile His Val Ile Thr Pro Asn
               660                 665                 670
   Lys Lys Ala Asn Ser Gly Pro Leu Asp Gln Tyr Leu Lys Leu Arg Asp
           675                 680                 685
   Leu Gln Arg Lys Ser Tyr Thr His Tyr Phe Tyr Glu Ala Thr Val Gly
       690                 695                 700
   Ala Gly Leu Pro Ile Ile Ser Thr Leu Arg Gly Leu Leu Glu Thr Gly
   705                 710                 715                 720
   Asp Lys Ile Leu Arg Ile Glu Gly Ile Cys Ser Gly Thr Leu Ser Tyr
                   725                 730                 735
   Leu Phe Asn Asn Phe Val Gly Asp Arg Ser Phe Ser Glu Val Val Thr
               740                 745                 750
   Glu Ala Lys Asn Ala Gly Phe Thr Glu Pro Asp Pro Arg Asp Asp Leu
           755                 760                 765
   Ser Gly Thr Asp Val Ala Arg Lys Val Ile Ile Leu Ala Arg Glu Ser
       770                 775                 780
   Gly Leu Lys Leu Asp Leu Ala Asp Leu Pro Ile Arg Ser Leu Val Pro
   785                 790                 795                 800
   Glu Pro Leu Lys Gly Cys Thr Ser Val Glu Glu Phe Met Glu Lys Leu
                   805                 810                 815
   Pro Gln Tyr Asp Gly Asp Leu Ala Lys Glu Arg Leu Asp Ala Glu Asn
               820                 825                 830
   Ser Gly Glu Val Leu Arg Tyr Val Gly Val Val Asp Ala Val Asn Gln
           835                 840                 845
   Lys Gly Thr Val Glu Leu Arg Arg Tyr Lys Lys Glu His Pro Phe Ala
       850                 855                 860
   Gln Leu Ala Gly Ser Asp Asn Ile Ile Ala Phe Thr Thr Thr Arg Tyr
   865                 870                 875                 880
   Lys Asp His Pro Leu Ile Val Arg Gly Pro Gly Ala Gly Ala Gln Val
                   885                 890                 895
   Thr Ala Gly Gly Ile Phe Ser Asp Ile Leu Arg Leu Ala Ser Tyr Leu
               900                 905                 910
   Gly Ala Pro Ser
           915
<210> 8
   <211> 26
   <212> DNA
   <213> Artificial
<220>
   <223> Aspartate kinase (At4g19710) wild type PCR primers (forward)
<400> 8
   atggcgactc tgaagccgtc atttac 26
<210> 9
   <211> 27
   <212> DNA
   <213> Artificial
<220>
   <223> Aspartate kinase (At4g19710) wild type PCR primers (reverse)
<400> 9
   ttaagacggt gcaccgagat aagatgc 27
<210> 10
   <211> 37
   <212> DNA
   <213> Artificial
<220>
   <223> Aspartate kinase (At4g19710) mutant PCR primers (Q443A) (forward)
<400> 10
   gtgattatga tatcagctgc tagcagtgag cattctg 37
<210> 11
   <211> 38
   <212> DNA
   <213> Artificial
<220>
   <223> Aspartate kinase (At4g19710) mutant PCR primers (Q443A) (reverse)
<400> 11
   cagaatgctc actgctagca gctgatatca taattcac 38
<210> 12
   <211> 37
   <212> DNA
   <213> Artificial
<220>
   <223> Aspartate kinase (At4g19710) mutant PCR primers (Q524A) (forward)
<400> 12
   gtccgagcta tatctgctgg ttgttctgag tacaatg 37
<210> 13
   <211> 37
   <212> DNA
   <213> Artificial
<220>
   <223> Aspartate kinase (At4g19710) mutant PCR primers (Q524A) (reverse)
<400> 13
   cattgatctc agaacaacca gcagatatag ctcggac 37